# NEUE EUROPÄISCHE PATENTSCHRIFT

(11) **EP 0 309 514 B2**
(45) Veröffentlichungstag und Bekanntmachung des Hinweises auf die Entscheidung über den Einspruch: **15.10.1997**
(45) Hinweis auf die Patenterteilung: 02.12.1992
(21) Anmeldenummer: 88902851.0
(22) Anmeldetag: 05.04.1988
(51) Int. Cl.: C09K 19/34, C07D 277/20, C07D 285/08, C07D 285/14

(54) **THIAZOL- UND THIADIAZOL-DERIVATE ENTHALTENDE MEDIEN MIT SMEKTISCHER FLÜSSIGKRISTALLINER PHASE**
MEDIA CONTAINING DERIVATIVES OF THIAZOLE AND THIADIAZOLE, WITH SMECTIC LIQUID-CRYSTAL PHASE
MILIEUX CONTENANT DES DERIVES DE THIAZOLE ET DE THIADIAZOLE A PHASE SMECTIQUE DE CRISTAUX LIQUIDES

(30) Priorität: 16.04.1987 DE 3712995; 15.09.1987 DE 3730859
(43) Veröffentlichungstag der Anmeldung: 05.04.1989
(73) Patentinhaber: MERCK PATENT GmbH, D-64271 Darmstadt (DE)
(72) Erfinder: GRAY, Georg, William, Cottingham N. Humberside HU16 4DY (GB); SCROWSTON, Richard, Michael, N. Humberside HU17 8TG (GB); TOYNE, Kenneth, Johnson, N. Humberside HU6 8QW (GB); LACEY, David, N. Humberside HU8 9NW (GB); JACKSON, Adam, N. Ferriby N. Humberside HU14 3QG (GB); KRAUSE, Joachim, D-6110 Dieburg (DE); POETSCH, Eike, D-6109 Mühltal (DE); GEELHAAR, Thomas, D-6500 Mainz (DE); WEBER, Georg, D-6106 Erzhausen (DE); WÄCHTLER, Andreas, D-6103 Griesheim (DE)
(86) Internationale Anmeldenummer: EP8800279
(87) Internationale Veröffentlichungsnummer: WO8808019

(56) Entgegenhaltungen:
- EP-A- 0 178 647
- WO-A-86/06401
- WO-A-86/07085
- DD-A- 117 014
- DE-A- 3 627 964
- DE-A- 3 703 651
- GB-A- 2 182 037
- Chemical Abstracts, vol. 92, 1980, (Columbus Ohio, US), K. Doelling et al.:"Liquid-crystals thiazoles", see page 653, abstract no. 110908j
- Mol. Cryst. Liq. Cryst. 1 (1985), S. 39-44
- J. f. Prakt. Chemie, 322 (1980), S. 933-944
- J. f. Prakt. Chemie, 321 (1979), S. 643-654

## Beschreibung

Die Erfindung betrifft die Verwendung von chiralen und/oder achiralen Verbindungen mit Thiazol- oder Thiadiazolstrukturelement als Komponenten in Medien mit smektischer flüssigkristalliner Phase. Die Erfindung betrifft auch Medien mit smektischer flüssigkristalliner Phase, insbesondere mit chiraler getilteter smektischer flüssigkristalliner Phase, die Verbindungen mit Thiazol- oder Thiadiazolstrukturelement enthalten.

Medien mit chiralen getilteten smektischen flüssigkristallinen Phasen und ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L-771 (1983). Solche Medien können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiralen getilteten Phase beruhen.

In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtung der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. S_{C}*, jedoch auch S_{H}*, S_{I}*, S_{J}*, S_{K}*, S_{G}*, S_{F}*) ist deren geringe chemische, thermische und Photo-Stabilität.

Eine weitere nachteilige Eigenschaft von Displays basierend auf derzeit verfügbaren chiralen getilteten smektischen Mischungen ist, daß die Spontanpolarisation zu kleine Werte aufweist und/oder die Viskosität zu hoch ist, so daß das Schaltzeitverhalten der Displays ungünstig beeinflußt wird und/oder der Pitch und der Tilt der Phasen nicht den Anforderungen der Display-Technologie entspricht. Darüber hinaus ist meist der Temperaturbereich der ferroelektrischen Phasen zu klein und liegt überwiegend bei zu hohen Temperaturen.

Es wurde nun gefunden, daß die Verwendung von Verbindungen der Formel I

R¹-A¹-Z¹-A²-(Z²-A³)ₙ-R² (I)

worin mindestens einer der Ringe A¹, A² und A³ die Gruppen Thiazol-2,4-diyl, Thiazol-2,5-diyl oder 1,3,4-Thiadiazol-2,5-diyl;
- A¹, A², und A³: ansonsten jeweils unabhängig voneinander eine unsubstituierte oder aurcn Halogen, Nitril und/oder Alkyl ein- oder mehrfach substituierte 1,4-Phenylengruppe, worin auch eine oder mehrere CH-Gruppen durch N ersetzt sein können;
eine 1,4-Cyclohexylengruppe, die gegebenenfalls mit CN substituiert sein kann; die Gruppen 1,4-Bicyclo-(2,2,2)-octylen, Piperidin-1,4-diyl, Naphthalin-2,6-diyl, Decahydronaphthalin-2,6-diyl oder 1,2,3,4-Tetrahydronaphthalin-2,6-diyl;
- R¹ und R²: jeweils unabhängig voneinander eine Alkylgruppe mit 1-15 C-Atomen;
ein hiervon abgeleiteter organischer Rest, der eine oder mehrere der Gruppen -O-, -S-, -CO-, -O-CO-, -CO-O-, -CO-S-, -S-CO-, -CHHalogen-, -CF₂-, -CHCN-, -CAlkylCN- und/oder -CH = CH-, -C≡C-enthalten und gegebenenfalls ein optische Aktivität verursachendes asymmetrisches Kohlenstoffatom aufweisen kann;
einer der Reste R¹ und R² auch F, Cl, Br, CN, COOH, OH, SH, NH₂, NO₂ oder -NCS;
- Z¹ und Z²: jeweils unabhängig voneinander -CO-O-, -OCO-, -CH₂CH₂-, -CH₂-O-, -OCH₂-, -N=N-, -NO = N-, -CH = N-oder eine Einfachbindung; eine der Gruppen Z¹ und Z² auch -CH₂-, -O-, -CO-, -CHCN-, -CHHalogen-, -CH₂CH₂CH₂-, -CH₂-COO- oder -CH₂OCO-
und
- n: 0, 1, 2 oder 3 bedeutet,
als Komponenten von Medien mit getilteter smektisch-flüssigkristalliner Phase mit den Maßgaben, daß die Phase 3-15 Komponenten enthält, daß die Phase keine Komponente enthält, bei der am Kohlenstoffatom in der 4-Position des terminalen aromatischen Rings ein H-Atom gebunden ist, und daß R¹ und R² in den Verbindungen der Formel I nicht 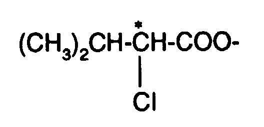 bedeuten,
die erwähnten Nachteile wesentlich vermindern kann.

Diese Verbindungen sind somit als Komponenten von Medien mit chiral getilteter smektischer flüssigkristalliner Phase vorzüglich geeignet. Insbesondere sind mit ihrer Hilfe chemisch besonders stabile Medien mit chiral getilteter smektischer flüssigkristallienr Phase und günstigen ferroelektrischen Phasenbereichen, insbesondere mit breiten S_{C}* Phasen bereichen, hervorragender Unterkühlbarkeit bis zu Temperaturen unter 0°C ohne das Kristallisation auftritt und für derartige Phasen hohe Werte für die spontane Polarisation (in nC/cm²) herstellbar.

Zwar sind Verbindungen mit Thiadiazolstrukturelement für den Einsatz in Flüssigkristallmaterialien bekannt, allerdings nur als nemtaische Flüssigkristalle (DD 117 014), als Bestandteile nematischer Flüssigkristallmischungen mit negativer dielektrischer Anisotropie (DE 37 03 651) oder als nicht flüssigkristalline Zusatzstoffe in nematischen Flüssigkristallmischungen (JP 50-92279). In Mol. Cryst. Liq. Cryst.. 1 (1985), 39-44 wird eine binäre Mischung von zwei 1,3,4-Thiadiazol-2,5-diyl-Derivaten mit einer S_{A}- bzw. S_{C}-Phase beschrieben. Der Schmelzpunkt bei der günstigsten Mischungszusammensetzung ist allerdings mit 72 °C so hoch, daß diese Mischung für praktische Anwendungen nicht in Betracht kommt.

Es sind jedoch in keinem der genannten Dokumente Hinweise zu finden, daß diese Verbindungen als Komponenten chiraler getilteter smektischer Mischungen für ferroelektrische Displays, basierend z.B. auf der von Clark und Lagerwall beschriebenen SSFLC-Technologie, verwendet werden können.

In DE-OS 36 27 964 werden chirale verzweigte α-Chlorcarbonsäurederivate als ferroelektrische Flüssigkristalle beschrieben. Die dort angegebene, sehr breit gefaßte allgemeine Formel kann auch Verbindungen umfassen, die das 1,3,4-Thiadiazol-2,5-diyl-Strukturelement enthalten. Es ist jedoch keine solche Verbindung konkret offenbart, so daß davon ausgegangen werden muß, daß der besondere Wert weder dieser speziellen Verbindungsklasse noch der von Verbindungen allgemein mit Thiazol- oder Thiadiazol-Strukturelement erkannt oder gar genutzt worden ist.

Gegenstand der Erfindung ist somit die Verwendung von chiralen und/oder achiralen Verbindungen der Formel I

R¹-A¹-Z¹-A²-(Z²-A³)ₙ-R² (I)

worin mindestens einer der Ringe A¹, A² und A³ die Gruppen Thiazol-2,4-diyl, Thiazol-2,5-diyl oder 1,3,4-Thiadiazol-2,5-diyl;
- A¹, A², und A³: ansonsten jeweils unabhängig voneinander eine unsubstituierte oder durch Halogen, Nitril und/oder Alkyl ein- oder mehrfach substituierte 1,4-Phenylengruppe, worin auch eine oder mehrere CH-Gruppen durch N ersetzt sein können;
eine 1,4-Cyclohexylengruppe, die gegebenenfalls mit CN substituiert sein kann; die Gruppen 1,4-Bicyclo-(2,2,2)-octylen, Piperidin-1,4-diyl, Naphthalin-2,6-diyl, Decahydronaphthalin-2,6-diyl oder 1,2,3,4-Tetrahydronaphthalin-2,6-diyl;
- R¹ und R²: jeweils unabhängig voneinander eine Alkylgruppe mit 1-15 C-Atomen;
ein hiervon abgeleiteter organischer Rest, der eine oder mehrere der Gruppen -O-, -S-, -CO-, -O-CO-, -CO-O-, -CO-S-, -S-CO-, -CHHalogen-, -CF₂-, -CHCN-, -CAlkylCN- und/oder -CH=CH-, -C≡C-enthalten und gegebenenfalls ein optische Aktivität verursachendes asymmetrisches Kohlenstoffatom aufweisen kann;
einer der Reste R¹ und R² auch F, Cl, Br, CN, COOH, OH, SH, NH₂, NO₂ oder -NCS;
- Z¹ und Z²: jeweils unabhängig voneinander -CO-O-, -OCO-, -CH₂CH₂-, -CH₂-O-, -OCH₂-, -N=N-, -NO=N-, -CH = N-oder eine Einfachbindung; eine der Gruppen Z¹ und Z² auch -CH₂-, -O-, -CO-, -CHCN-, -CHHalogen-, -CH₂CH₂CH₂-, -CH₂-COO- oder -CH₂OCO-
und
- n: 0, 1, 2 oder 3 bedeutet,
als Komponenten von Medien mit getilteter smektisch-flüssigkristalliner Phase mit den Maßgaben, daß die Phase 3-15 Komponenten enthält, daß die Phase keine Komponente enthält, bei der am Kohlenstoffatom in der 4-Position des terminalen aromatischen Rings ein H-Atom gebunden ist, und daß R¹ und R² in den Verbindungen der Formel I nicht 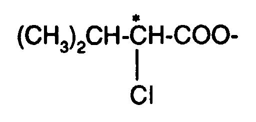 bedeuten.

Gegenstand der Erfindung sind auch solche Medien mit smektischer flüssigkristalliner Phase, die 3-15 Komponenten und dabei mindestens eine Verbindung der Formel I enthalten, sowie elektrooptische Anzeigeelemente mit solchen Medien als Dielektrikum.

Formel I umfaßt zum großen Teil auch neue Verbindungen. Es sind dies vornehmlich Verbindungen der Formel 1 mit den oben angegebenen Bedeutungen und der Maßgabe. daß mindestens einer der Reste R¹ und R² eine Alkylgruppe mit 4-15 C-Atomen ist, worin mindestens eine CH₂-Gruppe durch -CAlkylCN- ersetzt ist und ggf. eine oder mehrere weitere CH₂-Gruppen durch -O-, -S-, -CO-, -O-CO-, -CO-O-, -CO-S-, -S-CO-, -CHHalogen-, -CF₂-, -CHCN-, -CH = CH-, und/oder -C≡C- ersetzt sein können.

Gegenstand der Erfindung sind auch die neuen Verbindungen der Formel I. Es sind dies insbesondere Verbindungen der Formel I

R¹-A¹-Z¹-A²-(Z²-A³)ₙ-R² (I)

worin mindestens eienr der Ringe A¹, A² und A³ die Gruppen Thiazol-2,4-diyl, Thiazol-2,5-diyl oder 1,3,4-Thiadiazol-2,5-diyl,
- A¹, A², und A³: ansonsten jeweils unabhängig voneinander eine unsubstituierte oder durch Halogen, Nitril und/oder Alkyl ein- oder mehrfach substituierte 1,4-Phenylengruppe, worin auch eine oder mehrere CH-Gruppen durch N ersetzt sein können;
eine 1,4-Cyclohexylengrupe, die gegebenenfalls mit CN substituiert sein kann; die Grupen 1,4-Bicyclo-(2,2,2)-octylen, Piperidin-1,4-diyl, Naphthalin-2,6-diyl, Decahydronaphthalin-2,6-diyl oder 1,2,3,4-Tetrahydronaphthalin-2,6-diyl;
- R¹ und R²: jeweils unabhängig voneinander eine Alkylgruppe mit 1-15 C-Atomen;
ein hiervon abgeleiteter organischer Rest, der eine oder mehrere der Gruppen -O-, -S-, -CO-, -O-CO-, -CO-O-, -CO-S-, -S-CO-, -CHHalogen-, -CF₂-, -CHCN-, -CAlkylCN- und/oder -CH=CH-, -C≡C-enthalten und gegebenenfalls eine optische Aktivität verursachendes asymmetrisches Kohlenstoffatom aufweisen kann; wobei zwei Heteroatome nicht direkt miteinander verknüpft sind;
einer der Reste R¹ und R² auch F, Cl, Br, CN, COOH, OH, SH, NH₂, NO₂ oder -NCS;
- Z¹ und Z²: jeweils unabhängig voneinander -CO-O-, -OCO-, -CH₂CH₂-, -CH₂-O-, -OCH₂-, -N=N-, -NO = N-, -CH = N-oder eine Einfachbindung; eine der Gruppen Z¹ und Z² auch -CH₂-, -O-, -CO-, -CHCN-, -CHHalogen-, -CH₂CH₂CH₂-, -CH₂-COO- oder -CH₂OCO-
und
- n: 0, 1, 2 oder 3 bedeutet, mit der Maßgabe, daß mindestens einer der Reste R¹ und R² eine Alkylgruppe mit 4-15 C-Atomen ist, worin mindestens eine CH₂-Gruppe durch -CAlkylCN- ersetzt ist und ggf. eine oder mehrere weitere CH₂-Gruppen durch -O-, -S-, -CO-, -O-CO-, -CO-O-, -CO-S-, -S-CO-, -CHHalogen-, -CF₂-, -CHCN-, -CH = CH- und/oder -C≡C- ersetzt sein können.
Weiterhin gehören hierzu
Thiadiazol-Derivate der Formel 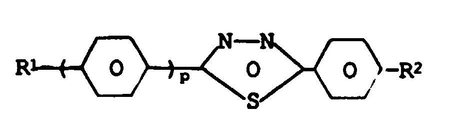 worin p 0 oder 1,
eine der Gruppen R¹ und R² 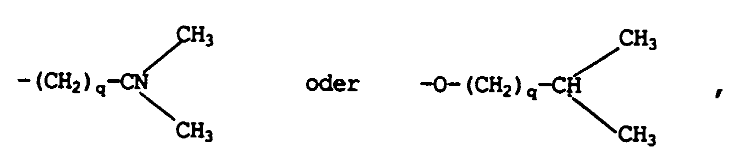 wobei q 2 bis 7, insbesondere 3 bedeuten kann, und die andere der Gruppen R¹ und R² geradkettiges Alkyl oder Alkoxy mit 3 bis 12 C-Atomen ist, sowie
Thiadiazol-Derivate der Formel 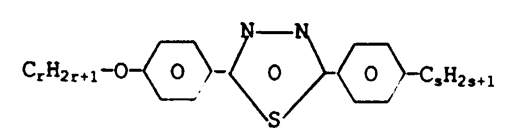 worin r 6, 8, 9 oder 10 und s 5, 6, 7, 8, 9, 10, 11 oder 12 bedeutet und die Gruppen CᵣH₂ᵣ₊₁ und CₛH₂ₛ₊₁ vorzugsweise geradkettige Alkylgruppen sind.

Die Verbindungen der Formel I besitzen einen breiten Anwendungsbereich. In Abhängigkeit von der Auswahl der Substituenten können diese Verbindungen flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder den Pitch und/oder den Phasenbereich eines solchen Dielektrikums zu varriieren, sie können aber auch als Basismaterialien dienen, aus denen flüssigkristalline Medien zum überwiegenden Teil zusammengesetzt sind.

Die Verbindungen der Formel I eignen sich ferner als Zwischenprodukte zur Herstellung anderer Substanzen, die sich als Bestandteile flüssigkristallines Dielektrika verwenden lassen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristallines Gemische eignen, erheblich verbreitert.

Gemäß den angegebenen Definitionen für R¹, R², A¹, A², A³, Z¹, Z² und n umfaßt Formel I Verbindungen mit 2 bis 5 Ringen, wovon mindestens einer ein Ringstrukturelement der Gruppe Thiazol-2,4-diyl, Thiazol-2,5-diyl oder 1,3,4-Thiadiazol-2,5-diyl ist.

Vorzugsweise ist nur einer der Ringe A¹, A² und A³ einer der vorbezeichneten Strukturelemente; bevorzugt ist dabei insbesondere 1,3,4-Thiadiazol-2,5-diyl.

Die Verbindungen der Formel I umfassen dementsprechend Verbindungen mit zwei Ringen der Teilformeln la und Ib:

R¹-A¹-A²-R² Ia

R¹-A¹-Z¹-A²-R² Ib,

Verbindungen mit drei Ringen der Teilformeln Ic bis le:

R¹-A¹-A²-A³-R² Ic

R¹-A¹-Z¹-A²-A³-R² Id

R¹-A¹-Z¹-A²-Z²-A³-R² Ie,

Verbindungen mit vier Ringen der Teilformeln If bis Ik:

R¹-A¹-A²-A³-A³-R² If

R¹-A¹-Z¹-A²-A³-A³-R² Ig

R¹-A¹-A²-Z²-A³-A³-R² Ih

R¹-A¹-Z¹-A²-Z²-A³-A³-R² Ii

R¹-A¹-Z¹-A²-A³-Z²-A³-R² Ij

R¹-A¹-Z¹-A²-Z²-A³-Z²-A³-R² Ik

sowie Verbindungen mit fünf Ringen der Teilformeln II bis It:

R¹-A¹-A²-A³-A³-A³-R² Il

R¹-A¹-Z¹-A²-A³-A³-A³-R² Im

R¹-A¹-A²-Z²-A³-A³-A³-R² In

R¹-A¹-Z¹-A²-Z²-A³-A³-A³-R² Io

R¹-A¹-Z¹-A²-A³-Z²-A³-A³-R² Ip

R¹-A¹-Z¹-A²-A³-A³-Z²-A³-R² Iq

R¹-A¹-Z¹-A²-Z²-A³-Z²-A³-A³-R² Ir

R¹-A¹-Z¹-A²-Z²-A³-A³-Z²-A³-R² Is

R¹-A¹-Z¹-A²-Z²-A³-Z²-A³-Z²-A³-R² It

Bevorzugte Verbindungen sind solche mit 2 oder 3 Ringen, insbesondere solche der Formeln la und Ic. Besonders bevorzugt sind zweikernige Verbindungen der Formel la.

Sofem A¹, A² und A³ nicht für einen der vorstehend angegebenen Thiazol- oder Thiadiazol-Ringstrukturelemente steht, können diese jeweils unabhängig voneinander eine unsubstituierte oder durch Halogen, Nitril und/oder Alkyl ein- oder mehrfach substituierte 1,4-Phenylengruppe, worin auch eine oder mehrere CH-Gruppen durch N ersetzt sein können, eine 1,4-Cyclohexylengruppe, die gegebenenfalls mit CN substituiert sein kann, oder die Gruppen 1,4-Bicyclo-(2,2,2)-octylen, Piperidin-1,4-diyl, Naphthalin-2,5-diyl, Decahydronaphthalin-2,6-diyl oder 1,2,3,4-Tetrahydronaphthalin-2,6-diyl bedeuten.

Der Einfachheit halber bedeuten im folgenden Cy eine 1,4-Cyclohexylengruppe, Bi eine Bicyclo[2,2,2]-octylengruppe, Pip eine Piperidin-1,5-diylgruppe, Phe eine 1,4-Phenylengruppe, Py eine Pyridin-2,5-diylgruppe, Pyr eine Pyrimidin-2,5-diylgruppe und Pyn eine Pyridazin-3,6-diylgruppe, wobei Phe und/oder Py und/oder Pyr und/oder Pyn unsubstituiert oder durch ein oder zwei F-und/oder Cl Atome und/oder CH₃-Gruppen und/oder CN-Gruppen substituiert sein können.

A¹, A² und A³ sind bevorzugt Cy, Phe, Py oder Pyr; bevorzugt enthalten die Verbindungen der Formel I nicht mehr als jeweils einen der Reste Pip, Bi, Pyn, Pyr.

R¹ und R² können sein jeweils unabhängig voneinander eine Alkylgruppe mit 1-15 C-Atomen, ein hiervon abgeleiteter organischer Rest, der eine oder mehrere Gruppen -O-, -S-, -CO-, -O-CO-, -CO-O-, -CO-S-, -S-CO-, -CHHalogen-, -CF₂-, -CHCN-, -CAlkylCN- und/oder -CH = CH- enthalten und gegebenenfalls ein optische Aktivität verursachendes asymmetrisches Kohlenstoffatom aufweisen kann, oder einer der Reste R¹ und R² auch F, Cl, Br, CM, COOH, OH, SH, NH₂, NO₂ oder -NCS.

R¹ und R² bedeuten vorzugsweise Alkyl oder Alkoxy, femer Polyfluoralkyl, jeweils mit 1-15 C-Atomen.

Weiterhin bevorzugt sind Verbindungen, in denen einer der Reste R¹, R² CN, -NCS, F oder Cl bedeutet.

Falls R¹ und R² Alkylreste und/oder Alkoxyreste bedeuten, so können sie geradkettig oder verzweigt sein. Vorzugsweise sind sie geradkettig, haben 1 bis 15, insbesondere 2 bis 12 C-Atome und bedeuten demnach bevorzugt Ethyl, Propyl, Butyl, Pentyl, Hexyl, Heptyl, Octyl, Nonyl, Decyl, Undecyl, Dodecyl, Ethoxy, Propoxy, Butoxy, Pentoxy, Hexoxy oder Heptoxy, Octoxy, Nonoxy, Decoxy, Undecoxy, Dodecoxy, ferner Methyl, Tridecyl, Tetradecyl, Pentadecyl, Methoxy, Tridecoxy, Tetradecoxy oder Pentadecoxy.

Besonders bevorzugte Verbindungen der Formel I sind solche, die unterschiedliche Flügelgruppen R¹ und R² im Molekül enthalten wie vorzugsweise Alkyl- und Alkoxygruppen. Verbindungen, die gleichzeitig Alkyl- und Alkoxy-Flügelgruppen enthalten, führen in ferroelektrischen flüssigkristallinen Mischungen zu deutlich niedrigeren Schmelzpunkten.

Verbindungen der Formel I sowie der vor- und nachstehenden Teilformeln mit verzweigten Flügelgruppen R¹ bzw. R² können gelegentlich wegen einer besseren Löslichkeit in den üblichen flüssigkristallinen Basismaterialien von Bedeutung sein, insbesondere aber als chirale Dotierstoffe für chirale getiltete smektische Phasen, wenn sie optisch aktiv sind. Hierzu ist erforderlich, daß die Reste R¹ bzw. R² über mindestens ein optische Aktivität verursachendes asymmetrisches Kohlenstoffatom verfügen.

Formel I umfaßt dann sowohl die Racemate dieser Verbindungen als auch die optischen Antipoden sowie deren Gemische. Solche Verbindungen eignen sich jedoch auch als Komponenten nematischer flüssigkristalliner Phasen, insbesondere zur Vermeidung von reverse twist. Verzweigte Gruppen dieser Art enthalten in der Regel eine oder zwei Kettenverzweigungen. Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, Cl oder Br), Alkyl oder Alkoxy mit jeweils 1 bis 5 C-Atomen und CN verknüpft. Der optisch aktive organische Rest R¹ bzw. R² hat vorzugsweise die Formel, 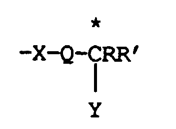 worin
- X: -CO-O-, -O-CO-, -O-CO-O-, -CO-, -O-, -S-, -CH≡CH-, -CH=CH-COO- oder eine Einfachbindung,
- Q: Alkylen mit 1 bis 5 C-Atomen, worin auch eine nicht mit X verknüpfte CH₂-Gruppe durch -O-, -CO-, -O-CO-, -CO-O- oder -CH=CH- ersetzt sein kann, oder eine Einfachbindung,
- Y: CN, Halogen, Methyl oder Methoxy, und
- R,R': voneinander und von Y verschieden jeweils H, eine Alkyl- oder Alkoxygruppe mit 1 bis 18 C-Atomen, worin auch eine oder zwei nicht benachbarte CH₂-Gruppen durch -O-, -CO-, -CO-, -CO-O- und/oder -CH-CH- ersetzt sein können,
bedeutet.
- X: ist vorzugsweise -CO-O-, -O-CO-, -CH=CH-OOO-(trans) oder eine Einfachbindung. Besonders bevorzugt sind -CO-O- und -O-CO/insbesondere -CO-O-.
- Q: ist vorzugsweise -CH₂-, -CH₂CH₂- oder eine Einfachbindung, insbesondere bevorzugt eine Einfachbindung.
- Y: ist vorzugsweise CH₃, -CN oder F, insbesondere bevorzuat -CN oder F.
- R,R': sind vorzugsweise verzweigt oder geradkettiges Alkyl mit 1 bis 10, insbesondere mit 1 bis 7, C-Atomen.

Bevorzugte verzweigte Reste R¹ und R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Chlorpropionyloxy, 2-Fluor-3-methylbutyryloxy, 2-Chlor-3-methylbutyryloxy, 2-Chlor-4-methylvaleryloxy, 2-Chlor-3-methylvaleryloxy, 2-Methyl-3-oxa-pentyl, 2-Methyl-3-oxa-hexyl, 2-Cyano-2-methylhexanoyloxy.

Verbindungen der Formel I, worin einer der Reste R¹ und R² die Formel -X-Q-CHCN-R (mit den oben angegebenen bevorzugten Bedeutungen) hat, können z.B. nach D.A. Evans und J.M. Takacs, Tetrahedron Lett. 21, 4233 (1980) hergestellt werden.

Polyfluoralkylgruppen, worin auch eine oder mehrere CF₂-Gruppen durch eine Gruppierung ausgewählt aus der Gruppe -O-, -S-, -CO-, -CH-Halogen-, -CHCN-, -O-CO-, -O-COO-, -CO-O- und -CH=CH- oder auch durch eine Kombination von zwei geeigneten Gruppierungen ersetzt sein können, wobei zwei Heteroatome nicht direkt miteinander verknüpft sind, bedeuten vorzugsweise Perfluoralkylgruppen mit 1 bis 15 C-Atomen, worin auch 1 bis 3 CH₂-Gruppen durch eine Gruppierung ausgewählt aus der Gruppe -O-, -CHHalogen-(insbesondere -CHF-), -O-CO-, -CO-O-und -O-COO- oder auch durch eine Kombination von zwei geeigneten Gruppierungen ersetzt sein können, wobei zwei Heteroatome nicht direkt miteinander verknüpft sind.

Besonders bevorzugte Gruppen sind diejenigen der Formeln R_{F},R_{F}CH₂, R_{F}CH₂CH₂, R_{F}CH₂O und R_{F}COO.

RF ist vorzugsweise eine geradkettige Perfluoralkylgruppe mit vorzugsweise 2 bis 12 C-Atomen, worin auch ein oder mehrere Fluoratome (vorzugsweise 1 oder 2 Fluoratome, vorzugsweise in ω- oder (ω-1)-Position) durch H ersetzt sein können.

Bevorzugte Verbindungen der Formel I, worin mindestens einer der Reste R¹ und R² eine Polyfluoralkylgruppe ist, führen zu erfindungsgemäßen Phase mit niedriger optischer Anisotropie und ausgeprägter S_{A}-Phase bei höheren Temperaturen.

Z¹ und Z² können jeweils unabhängig voneinander -CO-O-, -O-CO-, -CH₂CH₂-, -CH₂-O-, -OCH₂-, -N = N-, -NO = N-, -CH=N- oder eine Einfachbindung; eine der Gruppen Z¹ und Z² auch -CH₂-, -O-, -CO-, -CHCN-, CHHalogen-, -CH₂CH₂CH₂-, -CH₂-COO- oder -CH₂ OCO- bedeuten.

Z¹ und Z² sind bevorzugt Einfachbindungen, in zweiter Linie bevorzugt -CO-O-, -O-CO- oder -CH₂CH₂-Gruppen.

Besonders bevorzugt sind die folgenden kleineren Gruppen von Verbindungen, in denen -A- Thiazol-2,4-diyl, Thiazol-2,5-diyl oder 1,3,4-Thiadiazol-2,5-diyl bedeutet. Alkyl steht hier für Methyl, Ethyl, Propyl, Butyl und vorzugsweise für geradkettiges Pentyl, Hexyl, Heptyl, Octyl, Nonyl oder Decyl; Alkoxy bzw. Oxyalkoxy steht für Methoxy, Ethoxy, Propoxy, Butoxy sowie vorzugsweise für geradkettiges Pentoxy, Hexoxy, Heptoxy, Octoxy, Nonoxy oder Decoxy:
Alkyl-Phe-A-Alkyl
Alkoxy-Phe-A-Alkyl
Alkyl-OCO-Phe-A-Alkyl
Alkyl-Cyc-A-Alkyl
Alkoxy-Cyc-A-Alkyl
Alkyl-COO-Cyc-A-Alkyl
Alkyl-OCO-Cyc-A-Alkyl
Alkyl-Phe-Phe-A-Alkyl
Alkoxy-Phe-Phe-A-Alkyl
Alkyl-Phe-A-Phe-Alkyl
Alkoxy-Phe-A-Phe-Alkyl
Alkoxy-Phe-A-Phe-Alkoxy
Alkyl-Phe-Cyc-A-Alkyl
Alkyl-Phe-Phe-Cyc-A-Alkyl
Alkoxy-Phe-Cyc-A-Alkyl
Alkoxy-Phe-Phe-Cyc-A-Alkyl
Alkyl-Phe-CH₂CH₂-A-Alkyl
Alkyl-Phe-Phe-CH₂CH₂-A-Alkyl
Alkoxy-Phe-CH₂CH₂-A-Alkyl
Alkoxy-Phe-Phe-CH₂CH₂-A-Alkyl
Alkyl-Cyc-Cyc-A-Alkyl
Alkyl-Cyc-A-Phe-Alkyl
Alkoxy-Cyc-A-Phe-Alkyl
Alkoxy-Phe-A-Cyc-Alkyl
Alkyl-Py-A-Alkyl
Alkyl-Pyr-A-Alkyl
Alkyl-Cyc-COO-Phe-A-Alkyl
Die Verbindungen der Formel I werden nach an sich bekannten Methoden und unter den hierfür üblichen Reaktionsbedingungen hergestellt, wie sie in der Literatur (z.B. in den Standardwerken wie Houben-Weyl, Methoden der Organischen Chemie, Georg-Thieme-Verlag, Stuttgart), insbesondere in den dem einschlägigen Fachmann bekannten Publikationen zur Chemie flüssigkristalliner Verbindungen, beschrieben sind.

Die Einführung der Thiazol- bzw. Thiadiazol-Strukturelemente kann einerseits dadurch erfolgen, daß man Vorstufen, die diese Strukturelemente bereits enthalten nach den bekannten Methoden zu den Verbindungen der Formel I umsetzt. Andererseits können aber auch in entsprechend strukturierten Vorstufen oder Unterstruktureinheiten der Verbindungen der Formel I nach an sich bekannten Methoden Thiazol-bzw. Thiadiazol-Heterocyclenreste erzeugt werden.

So können beispielsweise 2,5-disubstituierte 1,3,4-Thiadiazole durch Umsetzung von N,N'-Diacylhydrazinen mit üblichen Thiierungsreagenzien wie P₄S₁₀ oder Lawesson's Reagenz hergestellt werden.

Die erfindungsgemäßen Medien mit smektischer flüssigkristalliner Phase bestehen aus 2 bis 25, vorzugsweise 3 bis 15 Komponenten, darunter mindestens einer Verbindung der Formel I. Die wichtigsten als Bestandteile derartiger flüssigkristalliner Medien in Frage kommenden Verbindungen lassen sich durch die Formel II charakterisieren,

R³-L-G-E-R⁴ (II)

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe, G ein Strukturelement aus der Gruppe

| | |
|---|---|
| -CH = CH- | -N(O)=N- |
| -CH = CY- | -CH = N(O)- |
| -C≡G- | -CH₂-CH₂- |
| -CO-O- | -CH₂-O- |
| -CO-S- | -CH₂-S- |
| -CH = N- | -COO-Phe-COO- |

oder eine C-C-Einfachbindung mit Y gleich Halogen, vorzugsweise Chlor, oder -CN, und R³ und R⁴ Alkyl, Alkoxy, Alkanoyloxy oder Alkoxycarbonyloxy mit bis zu 18, vorzugsweise bis zu 8 Kohlenstoffatomen, oder einer dieser Reste auch CN, NCS, NO₂, CF₃, F, Cl oder Br bedeuten.

Bei den meisten dieser Verbindungen sind R³ und R⁴ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Medien mit smektischer flüssigkristalliner Phase enthalten etwa 0,1 bis 99, vorzugsweise 10 bis 95 %, einer oder mehrerer Verbindungen der Formel I. Weiterhin bevorzugt sind flüssigkristalline Medien, die 0,1 bis 50, insbesondere 0,5 bis 30 % einer oder mehrerer Verbindungen der Formel I enthalten. Auch isotrope Verbindungen der Formel I können in den erfindungsgemäßen Medien verwendet werden.

Besonders bevorzugt sind erfindungsgemäße Medien mit chiraler getilteter smektischer flüssigkristalliner Phase, deren achirale Basismischung neben Verbindungen der Formel I mindestens eine andere Komponente mit betragsmäßig kleiner dielektrischer Anisotropie, niedriger Viskosität und breitem S_{c}-Phasenbereich enthalten. Diese weitere(n) Komponente(n) der achiralen Basismischung können beispielsweise 40 bis 90 %, vorzugsweise 50 bis 80 %, der Basismischung ausmachen. Als geeignete Komponenten kommen insbesondere Verbindungen der Teilformeln IIa und llb in Frage 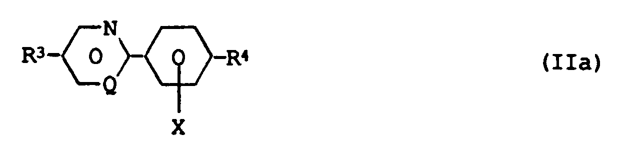 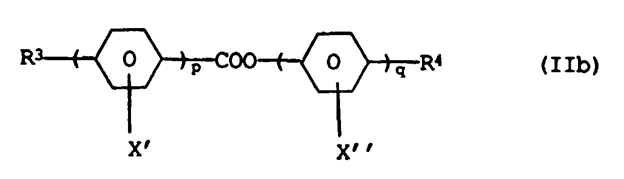 enthält, worin R³ und R⁴ jeweils unabhängig voneinander Alkyl oder Alkoxy mit 5 bis 12 C-Atomen, Q CH oder N und X, X' und X" jeweils unabhängig voneinander H oder F und p und q jeweils 1 oder 2 bedeuten, mit der Maßgabe, daß (p + q) 2 oder 3 ist.

Besonders bevorzugt sind Komponenten der Teilformel llc bis IIk: 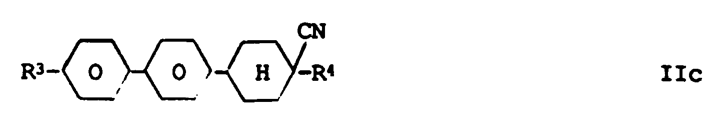 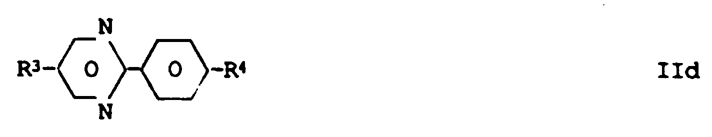 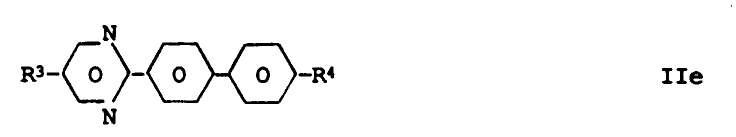 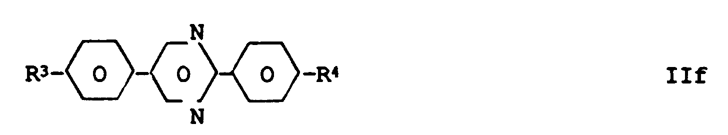 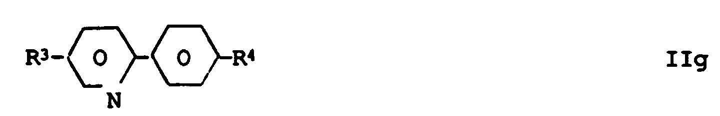 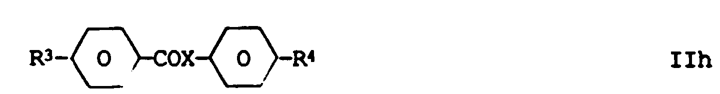 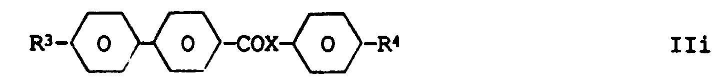 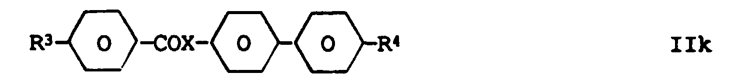 R³ und R⁴ sind jeweils vorzugsweise Alkyl, Alkoxy, Alkanoyloxy oder Alkoxycarbonyl mit jeweils 3 bis 12 C-Atomen. X ist O oder NH, vorzugsweise 0. In den Verbindungen der Formel IIc bis IIk kann auch eine 1,4-Phenylengruppe lateral durch Halogen, insbesondere bevorzugt durch Fluor, substituiert sein. Vorzugsweise ist eine der Gruppen R³ und R⁴ Alkyl und die andere Gruppe Alkoxy.

Besonders bevorzugt sind die Verbindungen der Teilformeln llc bis IIk, worin R³ und R⁴ jeweils geradkettiges Alkyl oder Alkoxy mit jeweils 5 bis 10 C-Atomen bedeutet.

Ferroelektrische Flüssigkristallmischungen, die Thiazol- und/oder Thiadiazolderivate enthalten, mit besonders günstigen Eigenschaften wie besonders niedrige Viskosität und besonders kurzer Schaltzeit, enthalten einen hohen Anteil, vorzugsweise 70 bis 80 %, an zweikernigen Substanzen mit smektischer Phase C sowie einen geringeren Anteil, vorzugsweise 10 bis 25 %, an dreikernigen Substanzen, die sich durch einen sehr hohen Übergang zwischen smektischer Phase C und A, zwischen smektischer Phase C und der nematischen oder isotropen Phase auszeichnen. Darüberhinaus enthalten solche Mischungen einen chiralen Dotierstoff, vorzugsweise in einem Anteil von 5 bis 15 %.

Derartige Mischungen weisen einen breiten Bereich der smektischen Phase C mit einem Übergang zur smektischen Phase A bei über 60 °C, eine bis zu 20 °C breite smektische Phase A, eine negative dielektrische Anisotropie, günstigen Tiltwinkel und sehr niedrige Viskositäten auf. Sie ermöglichen sehr schnelle Schaltzeiten (z.B. 30 µs bei Raumtemperatur).

Die Herstellung der erfindungsgemäßen Medien mit smektischer flüssigkristalliner Phase erfolgt in an sich üblicher Weise. In der Regel werden die Komponenten ineinander gelöst, zweckmäßig bei erhöhter Temperatur.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben. Es bedeuten ferner: K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

### Beispiel 1

Man suspendiert 31,3 g 4-Hydroxybenzoesäurehydrazid in 600 ml Pyridin, tropft bei 15-20 °C 208 ml Octansäurechlorid zu, erwärmt auf 100° und rührt noch 1 1/2 Stunden. Nach dem Abkühlen wird das Reaktionsgemisch in 3 1 Eis/Wasser gegossen, der Niederschlag abgesaugt, mit Wasser nachgewaschen und aus 2,2 l Methanol umkristallisiert. Man erhält 178 g N-(4-Octanoyloxybenzoyl)-N'-Octanoylhydrazin mit einem Schmelzpunkt von 131 °C.

Man löst 40,5 g dieser Verbindung unter Erwärmen in 500 ml THF, kühlt auf Raumtemperatur, gibt 44,5 g Lawesson's Reagenz zu und rührt noch 23 Stunden. Anschließend werden ca. 2/3 des THF abdestilliert, der Rückstand in 1 l Wasser und 100 ml 32%ige Natronlauge eingerührt, die ausgefallenen Kristalle abgesaugt, mit Wasser neutral gewaschen, getrocknet und aus Ethanol umkristallisiert. Man erhält 36,4 g 2-(4-n-Octanoyloxyphenyl)-5-n-heptyl-1,3,4-thiadiazol. K 78 S (77,5)l.

### Beispiel 2

30 g der vorstehend beschriebenen Verbindung, 9 g Natriumhydroxid, 104 ml Wasser und 200 ml Ethanol werden 2 Stunden unter Rückfluß erhitzt. Man destilliert das Ethanol weitgehend ab, verdünnt den Rückstand mit 500 ml Wasser, säuert an und stellt anschließend mit Natriumbicarbonat alkalisch. Man erhält 17,5 g 2-(4-Hydroxhenyl)-5-n-heptyl-1,3,4-thiadiazol mit einem Schmelzpunkt von 100 °C.

8,5 g der Hydroxyverbindung werden mit 2,5 ml Pyridin in 50 ml Toluol suspendiert und bei Raumtemperatur 6,7 g trans-4-n-Pentylcyclohexancarbonsäurechlorid zugetropft. Man rührt 2 Stunden bei 80 °C nach, saugt vom Pyridinhydrochlorid ab, wäscht die Toluolphase mit Wasser neutral und kristallisiert nach dem Abziehen des Lösungsmittels aus Ethanol um. Man erhält 10,5 g 2-[4-(trans-4-n-Pentylcyclohexanoyloxy)-phenyl]-5-n-heptyl-1,3,4-thiadiazol. K101 S 171 N 175l.

### Beispiel 3

46,8 g 4-n-Decyloxybenzoesäurehydrazid werden in 320 ml Pyridin gelöst. Bei Raumtemperatur tropft man 23,7 ml Heptafluorbuttersäurechlorid zu und rührt noch 1 1/2 Stunden nach. Anschließend gießt man auf 1600 ml Eis/Wasser, saugt die Kristalle ab und wäscht mit Wasser nach. Man kristallisiert aus Toluol um und erhält 39,9 g N-(4-n-Decyloxybenzoyl)-N'-heptafluorbutyrylhydrazin vom Schmelzpunkt 88 °C.

37,5 g dieser Verbindung und 34,3 g Lawesson's Reagenz werden im 385 ml THF 10 Stunden unter Rückfluß erhitzt. Man destilliert ca. 2/3 des Lösungsmittels ab, gießt den Rückstand in 800 ml Wasser und 80 ml 32%ige Natronlauge, saugt ab und kristallisiert den Niederschlag aus Ethanol unter Zusatz von Aktivkohle um.

Man erhält 30 g 2-(4-n-Decyloxyphenyl)-5-heptafluor-propyl-1,3,4-thiadiazol. K 70 l.

### Beispiel 4

8,64 g (S)-N-(4-n-Decyloxylbenzoyl)-N'-2-methyloctanoylhydrazin und 8,9 g Lawesson's Reagenz werden in 100 ml THF 5 Stunden unter Rückfluß erhitzt. Mach Aufarbeitun wie im vorhergehenden Beispiel erhält man 7,3 g (S)-2-(4-n-Decyloxyphenyl) -5-(1-methylheptyl)-1,3,4-thiadiazol.

### Beispiel 5

13,6 g (S)-2-Chlorisovaleriansäure, 1,35 g 4-Dimethylaminopyridin und 30,6 g 2-(4-Hydroxyphenyl)-5-n-nonyl-1,3,4-thiadiazol werden in 150 ml Dichlormethan vorgelegt, bei 10° unter Rühren eine Lösung von 21,9 g Dicyclohexylcarbodiimid in 30 ml Dichlormethan zugetropft und anschließend 15 Stunden bei Raumtemperatur nachgerührt. Man saugt über Kieselgel ab, verdampft das Lösungsmittel, kristallisiert den Rückstand aus Ethanol um und erhält 28,3 g (S)-2-[4-(2-Chlorisovaleroyloxy) phenyl]-5-n-nonyl-1,3,4-thiadiazol.

### Beispiel 6

Man löst 7,5 g N-(4-Heptyloxybenzoyl)-N'-octanoyl-hydrazin unter Erwärmen in 100 ml THF, kühlt auf 20 °C und fügt 8.9 g Lawesson's Reagenz hinzu. Nach siebzehnstündigem Rühren gießt man auf ein Gemisch aus 300 ml Eis/Wasser und 28 ml 32%iger Natronlauge. Die ausgefallenen Kristalle werden abgesaugt, mit Wasser gewaschen und aus 56 ml Ethanol umkristallisiert. Man erhält 6,5 g 2-(4-n-Heptanoyloxyphenyl)-5-n-heptyl-1,3,4-thiadiazol.

### Beispiele 7-64

Analog werden hergestellt:
2-(4-n-Octanoyloxyphenyl)-5-n-heptyl-1,3,4-thiadiazol K 78 S_{C} 79 I.
2-(4-n-Heptyloxyphenyl)-5-heptyl-1,3,4-thiadiazol K 79 S_{C} 89 I.
2-(4-n-Heptyloxyphenyl)-5-n-nonyl-1,3,4-thiadiazol H 77 S_{C} 89 I.
2-(4-n-Octyloxyphenyl)-5-n-pentyl-1,3,4-thiadiazol K 67 K 73 S_{C} (69) S_{A} 81 I.
2-(4-n-Octyloxyphenyl)-5-n-hexyl-1,3,4-thiadiazol K 73 S_{C} 80 S_{A} 83 I.
2-(4-n-Octyloxyphenyl)-5-n-heptyl-1,3,4-thiadiazol K 80 S_{C} 87 I.
2-(4-n-Decyloxyphenyl)-5-n-heptyl-1,3,4-thiadiazol K 70 S_{C} 89 I.
2-(4-n-Octyloxyphenyl)-5-(4'-n-heptylphenyl)-1,3,4-thiadiazol K 78 S_{C} 171 N 178 I.
2,5-Bis(4-n-Pentylphenyl)-1,3,4-thiadiazol K 93 S_{C} 123 N 164 I.
2,5-Bis(4-n-Heptylphenyl)-1,3,4-thiadiazol K 81 S_{C} 149 N 158 I.
2.5-Bis(4-n-Octyloxyphenyl)-1,3,4-thiadiazol K 101 S_{C} 194 N 196 I.
2,5-Bis(4-n-Nonyloxyphenyl)-1,3,4-thiadiazol K 93 S_{C} 187 I.
2-(4-n-Nonyloxyphenyl)-5-[4'-(2-methylbutyl)phenyl]-1,3,4-thiadiazol K 112 S_{C}* 133 N* 150 I.
2-(4-n-Nonyloxyphenyl)-5-[4'-(2-methylbutyloxy)phenyl]-1,3,4-thiadiazol K 90 S_{C}* 157 N* 163,5 I.
2-(4-n-Nonyloxyphenyl)-5-[4'-(2-methylbutyloxy)phenyl]-1,3,4-thiadiazol K 90 S_{C} 169 N 182 I.
2-(4-n-Nonyloxyphenyl)-5-(4'-Butyloxyphenyl)-1,3,4-thiadiazol K 97 S_{C} 179 N 201 I.
2-(2-Fluor-4-n-octyloxyphenyl)-5-(4'-n-nonyloxyphenyl)-1,3,4-thiadiazol K 94 S_{C} 130 S_{A} 165 N 178,7 I.
2-(2-Fluor-4-n-octyloxyphenyl)-5-[4'-(2-methylbutyl)-phenyl]-1,3,4-thiadiazol K 112 S_{C} 141 I.
2-n-Butyl-5-(4'-n-octyloxybiphenyl-4-yl)-1,3,4-thiadiazol K 126 S_{C} 149 S_{A} 209 I.
2-n-Pentyl-5-(4'-n-octyloxybiphenyl-4-yl)-1,3,4-thiadiazol K 134 S_{G} 146 S_{I} 158 S_{C} 194 S_{A} 212 I.
2-n-Nonyl-5-[4'-(3-methylbutyloxy)-biphenyl-4-yl]-1,3,4-thiadiazol K 75 S_{C} 126 I.
2-n-Nonyl-5-(2-fluor-4'-n-propylbiphenyl-4-yl)-1,3,4-thiadiazol K 84 S_{C} 93 N 123 I.
2-(4-n-Octyloxyphenyl)-5-(4'-n-pentylphenyl)-1,3,4-thiadiazol K 80 S_{C} 167 N 181,5 I.
2-(4-n-Octyloxyphenyl)-5-(4'-n-decylphenyl)-1,3,4-thiadiazol K 79 S_{C} 173 I.
r-1-Cyan-1-n-heptylcyclohexan-cis-4-carbonsäure-[p-(5-n-heptyl-1,3,4-thiadiazol-2-yl)]-phenylester K 73 S_{F} (66) S_{C} 77 S_{A} 167 I.
2-(4-n-Octyloxyphenyl)-5-n-octyl-1,3,4-thiadiazol K 80 S_{C} 90 I.
2-(4-n-Octyloxyphenyl)-5-n-nonyl-1,3,4-thiadiazol K 77 S_{C} 90 I.
2-(4-n-Decyloxyphenyl)-5-n-pentyl-1,3,4-thiadiazol K 73 S_{F} (55) S_{C} (57) S_{A} 84 I.
2-(4-n-Decyloxyphenyl)-5-n-nonyl-1,3,4-thiadiazol K 79 S_{C} 92 I.
2-(4-n-Hexyloxyphenyl)-5-(4'-n-pentylphenyl)-1,3,4-thiadiazol K 55 S_{C} 158 N 186 I.
2-(4-n-Octylphenyl)-5-(3'-n-pentylcyclopentyl-1,3,4-thiadiazol K 63 I.
2-[4-(3,7-Dimethyloctyloxy)-phenyl]-5-n-heptyl-1,3,4-thiadiazol K 48 I.
2-(4-n-Decyloxyphenyl)-5-(4'-n-heptylcyclohexyl)-1,3,4-thiadiazol K 97 S_{C} 146,6 S_{A} 170 I.
2-(4-n-Hexyloxyphenyl)-5-(4'-n-propylcyclohexyl)-1,3,4-thiadiazol K 59 S_{C} (56) S_{A} 119 N 143,9 I.
2-[4-(2-n-Heptyloxypropionyloxy)-phenyl]-5-(4'-n-octyloxyphenyl)-1,3,4-thiadiazol K 87 S_{C}* 141 I.
2-[4-(2-Methylbutyroyloxy)-phenyl]-5-(4'n-octyloxhenyl)-1,3,4-thiadiazol K 93 S_{C} 163 I.
2-[4-(2-Chlor-3-methylbutyroyloxyphenyl]-5-(4'-n-octyloxyphenyl)-1,3,4-thiadiazol K 112 S_{C}* 166 Ch 170,7 I.
2-(4-n-Decyloxyphenyl)-5-[4'-(4-methylpentyl)-phenyl]-1,3,4-thiadiazol K 79 K 87 S_{C} 157 N 160,2 I.
2-(4-n-Hexylphenyl)-5-(4'-n-decylphenyl)-1,3,4-thiadiazol K 66 S_{C} 168 N 172,9 I.
2-(4-n-Octyloxyphenyl)-5-(4'-perfluorhexylphenyl)-1,3,4-thiadiazol K 178 S_{C} 187 S_{A} 217 I.
2-(4-n-Octyloxyphenyl)-5-n-decyl-1,3,4-thiadiazol K 78 S_{G} (70) S_{C} 90 I.
2-(4-n-Nonyloxyphenyl)-5-n-hexyl-1,3,4-thiadiazol K 62 S_{G} (61) S_{C} 81 S_{A} 83 I.
2-(4-n-Nonyloxyphenyl)-5-n-nonyl-1,3,4-thiadiazol K 76 S_{C} 90 I.
2-(4-n-Decyloxyphenyl)-5-n-hexyl-1,3,4-thiadiazol K 62 S_{C} 80 S_{A} 84 I.
2-(4-n-Heptyloxyphenyl)-5-n-decyl-1,3,4-thiadiazol K 75 S_{C} 86 I.
2-(4-n-Nonyloxyphenyl)-5-n-pentyl-1,3,4-thiadiazol K 69 S_{G} (53) S_{C} 66 S_{A} 82 I.
2-(4-n-Heptyloxyphenyl)-5-n-pentyl-1,3,4-thiadiazol K 72 S_{C} 74 S_{A} 79 I.
2-(4-n-Heptyloxyphenyl)-5-n-hexyl-1,3,4-thiadiazol K 74 S_{C} 81 I.
2-(4-n-Heptyloxyphenyl)-5-n-octyl-1,3,4-thiadiazol K 70 S_{C} 85 I.
2-(4-n-Nonyloxyphenyl)-5-n-heptyl-1,3,4-thiadiazol K 72 S_{C} 87 I.
2-[4-(4-Methylpentyl)-phenyl]-5-(4'n-hexyloxyphenyl-1,3,4-thiadiazol K 89 S_{C} 154 N 169 I.
2-[4-(4-Methylpentyl)-phenyl]-5-(4'-n-heptyloxyphenyl-1,3,4-thiadiazol K 89 S_{C} 156 N I.
2-[4-(4-Methylbutyl)-phenyl]-5-(4'-n-decylphenyl)-1,3,4-thiadiazol K 92 S_{C} 140 N 153,1 I.

### Beispiel 65

Ein flüssigkristallines Medium bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin |
| 5 % | 2-p-Hexyloxyphenyl-5-nonylopyrimidin, |
| 20 % | 2-p-Nonyloxyphenyl-5-nonylpyrimidin, |
| 20 % | r-1-Cyan-cis-4-(4'-octyloxybiphenyl-4-yl)-1-octylcyclohexan, |
| 10 % | r-1-Cyan-cis-4-(4'-octyloxybiphenyl-4-yl)-1-nonylcyclohexan, |
| 9 % | r-1-Cyan-cis-4-(4'-heptyloxybiphenyl-4-yl)-1-hexylcylohexan, |
| 10 % | optisch aktives 4-(5-Heptylpyrimidyl-2-)-phenyl-2-chlorisovalerianat, |
| 4 % | 2-(4-n-Octyloxyphenyl)-5-n-hexyl-1,3,4-thiadiazol, |
| 6 % | 2-(4-n-Nonyloxyphenyl)-5-n-heptyl-1,3,4-thiadiazol, |
| 4 % | optisch aktives 2-[4-(2-Chlorisovaleroyloxy)phenyl]-5-n-heptyl-1,3,4-thiadiazol |

zeigt S_{C}* 64 S_{A} 80 Ch 83 I und eine spontane Polarisation von 17 nC/cm² bei 20°.

### Beispiel 66

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 3 % | 2-p-Hexyloxyphenyl-5-nonylpyrimidin, |
| 25 % | 2-p-Nonyloxyphenyl-5-nonylopyrimidin, |
| 15 % | r-1-Cyan-cis-4-(4'-octyloxybiphenyl-4-yl)-1-octylcyclohexan, |
| 20 % | r-1-Cyan-cis-4-(4'-octyloxybiphenyl-4-yl)-1-nonylcyclohexan, |
| 10 % | r-1-Cyan-cis-4-(4'-heptyloxybiphenyl-4-yl)-1-hexylcyclohexan, |
| 10 % | optisch aktives 4-(5-Heptylpyrimidyl-2-)-phenyl-2-chlorisovalerat, |
| 5 % | optisch aktives 2-[4-(2-Methyl-2-cyanisovaleroyloxy)-phenyl]-5-n-heptyl-1,3,4-thiadiazol |

zeigt S_{C}* 64 S_{A} 74 Ch 85 I und eine spontane Polarisation von 21 nC/cm² bei 20°.

### Beispiel 67

Man stellt eine flüssigkristalline Phase her bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 5 % | 2-p-Hexyloxyphenyl-5-nonylpyrimidin, |
| 25 % | 2-p-Nonyloxyphenyl-5-nonylpyrimidin, |
| 28 % | r-1-Cyan-cis-4-(4'-butyloxybiphenyl-4-yl)-1-octylcyclohexan, |
| 14 % | r-1-Cyan-cis-4-(trans-4-pentylcyclohexyl)-1-(trans-4-pentylcyclohexyl)-cyclohexan, |
| 10 % | 3-Methyl-2-chlorpentansäure-p-(5-n-octylpyridin-2-yl)-phenylester, |
| 5 % | 2-(4-n-Octyloxhenyl)-5-(4'-n-heptylphenyl)-1,3,4-thiadiazol. |

### Beispiel 68

| | |
|---|---|
| 2 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 6 % | 2-p-Hexyloxyphenyl-5-nonylpyrimidin, |
| 22 % | 2-p-Nonyloxyphenyl-5-nonylpyrimidin, |
| 15 % | r-1-Cyan-cis-4-(4'-octyloxybiphenyl-4-yl)-1-octylcyclohexan, |
| 8 % | 2-p-Heptyloxyphenyl-5-nonylpyridin, |
| 5 % | 2-(4-n-Octyloxyphenyl)-5-n-heptyl-1,3,4-thiadiazol, |
| 5 % | 2-(4-n-Hexyloxyphenyl)-5-n-octyl-1,3,4-thiadiazol, |
| 10 % | optisch aktives 4-(5-Heptylpyrimidyl-2-)-phenyl-2-chlorisovalerat, |
| 9 % | 2-(4-n-Pentyloxy)-5-(4'-n-octylphenyl)-1,3,4-thiadiazol, |
| 9 % | 2-(3-n-Heptyloxy)-5-(4'-n-octylphenyl)-1,3,4-thiadiazol |

zeigt S_{C}* 78 S_{A} 84 Ch 95 I und eine Schaltzeit von 75 µs bei Raumtemperatur.

### Beispiel 69

| | |
|---|---|
| 5 % | 2-p-Hexyloxyphenyl-5-heptylpyrimidin, |
| 5 % | 2-p-Heptyloxyphenyl-5-heptylpyrimidin, |
| 5 % | 2-p-Octyloxyphenyl-5-heptylpyrimidin, |
| 5 % | 2-p-Nonyloxyphenyl-5-heptylpyrimidin, |
| 6 % | 2-p-Hexyloxyphenyl-5-nonylpyrimidin, |
| 22 % | 2-p-Nonyloxyphenyl-5-nonylpyrimidin, |
| 10 % | optisch aktives 4-(5-Heptylpyrimidyl-2-)-phenyl-2-chlorisovalerat, |
| 9 % | 2-(4-n-Hexyloxyphenyl)-5-(4'-n-octylphenyl)-1,3,4-thiadiazol, |
| 9 % | 2-(4-n-Octyloxhenyl)-5-(4'-heptylphenyl)-1,3,4-thiadiazol, |
| 12 % | 2-(4-n-Pentyloxy)-5-(4'-n-octylphenyl)-1,3,4-thiadiazol, |
| 12 % | 2-(4-n-Heptyloxy)-5-(4'-n-octylphenyl)-1,3,4-thiadiazol |

zeigt S_{C}* 68 S_{A} 85 I und eine Schaltzeit von 30 µs bei Raumtemperatur.

### Beispiel 70

| | |
|---|---|
| 5 % | 2-p-Hexyloxyphenyl-5-heptylpyrimidin, |
| 5 % | 2-p-Heptyloxyphenyl-5-heptylpyrimidin, |
| 5 % | 2-p-Octyloxyphenyl-5-heptylpyrimidin, |
| 5 % | 2-p-Monyloxyphenol-5-heptylpyrimidin, |
| 7 % | 2-p-Hexyloxyphenyl-5-nonylpyrimidin, |
| 24 % | 2-p-Nonyloxyphenyl-5-nonylpyrimidin, |
| 9 % | 2-p-Heptyloxyphenyl-5-nonylpyrimidin, |
| 8 % | 2-p-Octyloxyphenyl-5-nonylpyrimidin, |
| 10 % | r-1-Cyan-cis-4-(4'-octyloxybiphenyl-4-yl)-1-octylcyclohexan, |
| 10 % | optisch aktives 4-(5-Heptylpyrimidyl-2-)-phenyl-2-chlorisovalerat, |
| 6 % | 2-(4-n-Pentyloxyphenyl)-5-(4'-n-octylphenyl)-1,3,4-thiadiazol, |
| 6 % | 2-(4-n-Heptyloxyphenyl)-5-(4'-n-octylphenyl)-1,3,4-thiadiazol |

zeigt S_{C}* 65 S_{A} 74 Ch 83 I und eine Schaltzeit von 50 µs bei Raumtemperatur.

### Beispiel 71

| | |
|---|---|
| 4 % | 2-p-Hexyloxyphenyl-5-heptylpyrimidin, |
| 4 % | 2-p-Heptyloxyphenyl-5-heptylpyrimidin, |
| 4 % | 2-p-Octyloxyphenyl-5-heptylpyrimidin, |
| 5 % | 2-p-Nonyloxyphenyl-5-heptylpyrimidin, |
| 7 % | 2-p-Hexyloxyphenyl-5-nonylpyrimidin, |
| 24 % | 2-p-Nonyloxyphenyl-5-nonylpyrimidin, |
| 4 % | 2-p-Heptyloxyphenyl-5-nonylpyrimidin, |
| 5 % | 2-p-Octyloxyphenyl-5-nonylpyrimidin, |
| 9 % | r-1-Cyan-cis-(4'-octyloxybiphenyl-4-yl)-1-octylcyclohexan, |
| 16 % | optisch aktives 4-(5-Heptylpyrimidyl-2-)-phenyl-2-chlorisovalerat, |
| 9 % | 2-(4-n-Pentyloxyphenyl)-5-(4'-n-octylphenyl)-1,3,4-thiadiazol, |
| 9 % | 2-(4-n-Heptyloxyphenyl)-5-(4'-n-octylphenyl)-1,3,4-thiadiazol, |
| 1 % | optisch aktives 2-p-(2,6-Dimethylheptyloxy)-phenyl-5-nonylpyrimidin |

zeigt K 20 S_{C}* 62 S_{A}* 77 Ch 95 I.

### Beispiel 72

Unter Feuchtigkeitsausschluß wird zu einem Gemisch aus 0,1 mol 2-Heptyl-5-p-hydroxyphenyl-1,3,4-thiadiazol und 0,1 mol optisch aktiver 2-Methyl-2-butylcyanessigsäure in 200 ml Dichlormethan bei Eiskühlung 0,1 mol Dicyclohexylcarbodiimid in Dichlormethan zugegeben. Man rührt noch 12 Stunden bei Raumtemperatur, filtriert von Dicyclohexylharnstoff ab und isoliert durch übliche Aufarbeitung 2-[4-(2-Cyan-2-methylhexanoyloxy)-phenyl]-5-n-heptyl-1,3,4-thiadiazol.

### Beispiel 73

Zu 18,1 g p-Heptyloxybenzoesäurehydrazid in 150 ml Pyridin gibt man 13,3 g optisch aktives 2-Methyl-2-butylcyanessigsäurechlorid und rührt etwa 12 Stunden bei Raumtemperatur. Danach gießt man das Reaktionsgemisch in Wasser, extrahiert mit Dichlormethan und isoliert das Reaktionsprodukt durch Eindampfen und Umkristallisation aus Toluol/Hexan.

11,2 g des so erhaltenen Produktes werden zusammen mit 12,1 g Lawesson's Reagenz in 150 ml THF 3 Stunden unter Rückfluß gekocht. Danach wird in wäßrige Natronlauge eingerührt. Durch übliche Aufarbeitung der organischen Phase erhält man 2-(1-Cyan-1-methylpentyl)-5-(4-hexyloxyphenyl)-1,3,4-thiadiazol.

### Beispiel 74

| | |
|---|---|
| 8 % | 4-n-Octyloxybenzoesäure-4'-n-heptyloxyphenylester |
| 10 % | 4-n-Octyloxybenzoesäure-4'-n-octyloxyphenylester |
| 12 % | 4-n-Octyloxybenzoesäure-4'-n-nonyloxhenylester |
| 7 % | 4-n-Decyloxybenzoesäure-4'-n-hexyloxyphenylester |
| 9 % | 4-n-Decyloxybenzoesäure-4'-n-heptyloxyphenylester |
| 11 % | 4-n-Decyloxybenzoesäure-4'-n-octyloxyphenylester |
| 7 % | 2-(4-n-Decyloxyphenyl)-5-n-heptyl-1,3,4-thiadiazol |
| 7 % | 2-(4-n-Decyloxyphenyl)-5-n-nonyl-1,3,4-thiadiazol |
| 9 % | 2-(4-n-Octyloxyphenyl)-5-n-(4'-n-pentylphenyl)-1,3,4-thiadiazol |
| 8 % | 2-(4-n-Octyloxhenyl)-5-n-(4'-n-heptylphenyl)-1,3,4-thiadiazol |
| 12 % | optisch aktives 4-(5-Heptylpyrimidyl-2-)phenyl-2-chlorisovalerat |

zeigt S_{C}* 62 S_{A} 66 Ch 76 I und eine Spontanpolarisation von 14 nC ^{•} cm⁻² bei 20 °C.

### Beispiel 75

| | |
|---|---|
| 8 % | 4-n-Octyloxybenzoesäure-4'-octyloxyphenylester |
| 11 % | 4-n-Octyloxybenzoesäure-4'-nonyloxyphenylester |
| 10 % | 4-n-Decyloxybenzoesäure-4'-heptyloxyphenylester |
| 12 % | 4-n-Decyloxybenzoesäure-4'-octyloxyphenylester |
| 8 % | 2-p-Hexyloxyphenyl-5-octylpyrimidin |
| 10 % | 2-p-Heptyloxyphenyl-5-octylpyrimidin |
| 12 % | 2-p-Octyloxyphenyl-5-octylpyrimidin |
| 9 % | 2-(4-n-Octyloxyphenyl)-5(4'-n-pentylphenyl-1,3,4-thiadiazol |
| 9 % | 2-(4-n-Decyloxyphenyl)-5-(4'-n-pentylphenyl)-1,3,4-thiadiazol |
| 10 % | optisch aktives 4-(5-Heptylpyrimidyl-2-)phenyl-2-chlorisovalerat |

zeigt S_{C}* 60 S_{A} 68 Ch 74 I und eine Spontanpolarisation von 12 nC ^{•} cm⁻² bei 20 °C.

### Beispiel 76

| | |
|---|---|
| 3 % | 2-p-Hexyloxyphenyl-5-heptylpyrimidin |
| 3 % | 2-p-Heptyloxyphenyl-5-heptylpyrimidin |
| 3 % | 2-p-Octyloxyphenyl-5-heptylpyrimidin |
| 3 % | 2-p-Nonyloxyphenyl-5-heptylpyrimidin |
| 7 % | 2-p-Hexyloxyphenyl-5-nonylpyrimidin |
| 23 % | 2-p-Nonyloxyphenyl-5-nonylpyrimidin |
| 28 % | r-1-Cyan-cis-4-(4'-butyloxybiphenyl-4-yl)-1-octylcyclohexan |
| 6 % | r-1-Cyan-cis-4-(trans-4-pentylcyclohexyl)-1-(trans-4-pentylcyclohexyl)-cyclohexan |
| 14 % | r-1-Cyan-cis-4-(4'-heptylbiphenyl-4-yl)-1-hexylcyclohexan |
| 10 % | 2-[4-(2-Methyl-2-cyanhexanoyloxy)phenyl]-5-n-heptyl-1,3,4-thiadiazol |

zeigt S_{C}* 63 S_{A} 71 Ch 86 I und eine Spontanpolarisation von 24 nC ^{•} cm⁻² bei 20 °C.

### Beispiel 77

| | |
|---|---|
| 3 % | 2-p-Hexyloxyphenyl-5-heptylpyrimidin |
| 3 % | 2-p-Heptyloxyphenyl-5-heptylpyrimidin |
| 3 % | 2-p-Octyloxyphenyl-5-heptylpyrimidin |
| 3 % | 2-p-Nonyloxyphenyl-5-heptylpyrimidin |
| 7 % | 2-p-Hexyloxyphenyl-5-nonylpyrimidin |
| 23 % | 2-p-Nonyloxyphenyl-5-nonylpyrimidin |
| 28 % | r-1-Cyan-cis-4-(4'-butyloxybiphenyl-4-yl)-1-octylcyclohexan |
| 6 % | r-1-Cyan-cis-4-(trans-4-pentylcyclohexyl)-1-(trans-4-pentylcyclohexyl)-cyclohexan |
| 14 % | r-1-Cyan-cis-4-(4'-heptylbiphenyl-4-yl)-1-hexylcyclohexan |
| 10 % | 2-[4-(2-Hexyloxyphenyl)-5-(2-methyl-2-cyanpentyl)-1,3,4-thiadiazol |

zeigt S_{C}* 45 S_{A} 64 Ch 83 I und eine Spontanpolarisation von 16 nC ^{•} cm⁻² bei 20 °C.

## Patentansprüche

1. Verwendung von Verbindungen der Formel I
R¹-A¹-Z¹-A²-(Z²-A³)ₙ-R² (I)
worin mindestens einer der Ringe A¹, A² und A³ die Gruppen 1,3-Thiazol-2,4-diyl, 1,3-Thiazol-2,5-diyl oder 1,3,4-Thiadiazol-2,5-diyl;
A¹, A² und A³ ansonsten jeweils unabhängig voneinander eine unsubstituierte oder durch Halogen, Nitril und/oder Alkyl ein- oder mehrfachsubstituierte 1,4-Phenylengruppe, worin auch eine oder mehrere CH-Gruppen durch N ersetzt sein können;
eine 1,4-Cyclohexylengruppe, die gegebenenfalls mit CN substituiert sein kann; die Gruppen 1,4-Bicyclo-(2,2,2)-octylen, Piperidin-1,4-diyl, Naphthalin -2,6-diyl, Decahydronaphthalin-2,6-diyl oder 1,2,3,4-Tetrahydronaphthalin-2,6-diyl;
R¹ und R² jeweils unabhängig voneinander eine Alkylgruppe mit 1-15 C-Atomen;
ein hiervon abgeleiteter organischer Rest, der eine oder mehrere der Gruppen -O-, -S-, -CO-, -O-CO-, -CO-O-, -CO-S-, -S-CO-, -CHHalogen-, -CF₂-, -CHCN-, -CAlkylCN- und/oder -CH=CH-, -C≡C- enthalten und gegebenenfalls ein optische Aktivität verursachendes asymmetrisches Kohlenstoffatom aufweisen kann; einer der Reste R¹ und R² auch F, Cl, Br, CN, COOH, OH, SH, NH₂, NO₂ oder -NCS;
Z¹ und Z² jeweils unabhängig voneinander -CO-O-, -OCO-, -CH₂CH₂-, -CH₂-O-, -OCH₂, -N=N-, -NO=N-, -CH=N-oder eine Einfachbindung; eine der Gruppen Z¹ und Z² auch -CH₂-, -O-, -CO-, -CHCN-, -CHHalogen-, -CH₂CH₂CH₂-, -CH₂-COO- oder CH₂OCO-
und
n 0, 1, 2 oder 3 bedeutet,
als Komponenten von Medien mit getilteter smektisch-flüssigkristalliner Phase mit den Maßgaben, daß die Phase 3-15 Komponenten enthält, daß die Phase keine Komponente enthält, bei der am Kohlenstoffatom in der 4-Position des terminalen aromatischen Rings ein H-Atom gebunden ist, und daß R¹ und R² in den Verbindungen der Formel I nicht 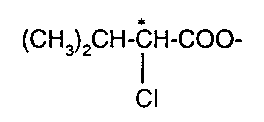 bedeuten.

2. Verwendung von Verbindungen der Formel I nach Anspruch 1 als Komponenten von Medien mit chiraler getilteter smektisch-flüssigkristalliner Phase gemäß Anspruch 1.

3. Verwendung nach Anspruch 1 oder 2 von Verbindungen der Formel I mit Ausnahme von dreikernigen 1,3,4-Thiadiazol-2,5-diyl-Derivaten, in denen eine der Flügelgruppen R¹ und R² ein chiraler Rest ist oder beide gleichzeitig eine Alkoxygruppe darstellen.

4. Medium, welches eine getiltete smektisch-flüssigkristalline Phase mit 3-15 Komponenten aufweist, von denen mindestens eine eine Verbindung der Formel ist, wobei R¹ und R² in den Verbindungen der Formel I nicht 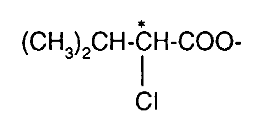 bedeuten, mit der Maßgabe, daß das Medium keine Komponente enthält, bei der am Kohlenstoffatom in der 4-Position des terminalen aromatischen Rings ein H-Atom gebunden ist.

5. Medium, eine chirale getiltete smektische flüssigkristalline Phase gemäß Anspruch 1 aufweisend, mit mindestens zwei Komponenten, dadurch gekennzeichnet, daß dieses mindestens eine Verbindung der Formel I enthält.

6. Medium, eine chirale getiltete smektisch-C-Phase gemäß Anspruch 1 aufweisend, mit mindestens zwei Komponenten, dadurch gekennzeichnet, daß diese mindestens eine Verbindung der Formel I enthält.

7. Medium, nach einem der Ansprüche 4-6, enthaltend mindestens eine 3- oder 4-kernige Verbindung der Formel I, worin eine der Brücken Z¹ und Z² eine Einfachbindung und der andere -COO-, -OCO- oder CH₂O- bedeutet.

8. Medium nach einem der Ansprüche 4-7, enthaltend mindestens eine Verbindung der Formel 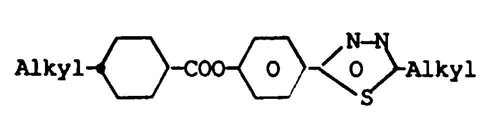 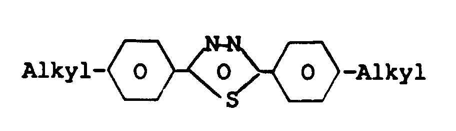 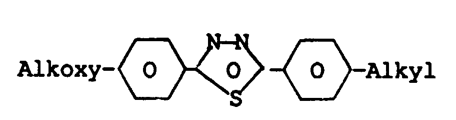 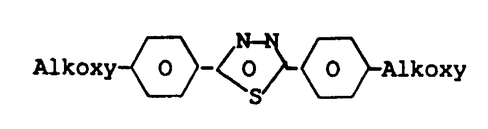 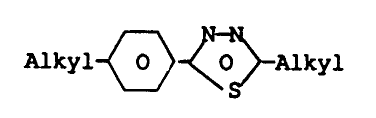 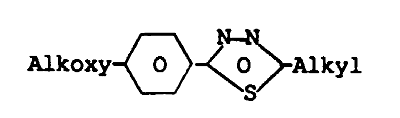 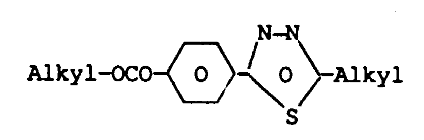 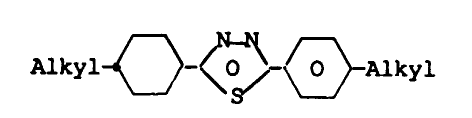 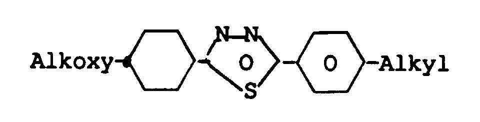 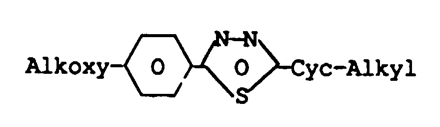 worin
Alkyl Methyl, Ehtyl, Propyl, Butyl, Pentyl, Hexyl, Heptyl, Octyl, Nonyl oder Decyl und
Alkoxy Methoxy, Ethoxy, Propoxy, Butixy, Pentixy, Hexoxy, Heptixy, Octixy, Nonoxy oder Decoxy
bedeuten.

9. Medium nach einem der Ansprüche 4-8, enthaltend mindestens eine Verbindung der Formel I, worin einer der Reste R¹ oder R² verzweigt ist und durch 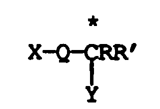 gegeben ist, worin
X -CO-O-, -O-CO-, -O-CO-O-, -CO-, -O-, -S-, -CH = CH-, -CH=CH-COO- oder eine Einfachbindung.
Q Alkylen mit 1 bis 5 C-Atomen, worin auch eine nicht mit X verknüpfte CH₂-Gruppe durch -O-, -CO-, -O-CO-, -CO-O- oder -CH = CH- ersetzt sein kann, oder eine Einfachbindung,
Y CN, Halogen, Methyl oder Methoxy, und
R, R' voneinander und von Y verschieden jeweils H, eine Alkyl- oder Alkoxygruppe mit 1 bis 18 C-Atomen, worin auch eine oder zwei nicht benachbarte CH₂-Gruppen durch -O-, -CO-, -O- CO-, -CO-O- und/oder -CH = CH- ersetzt sein können,
bedeuten.

10. Medium nach Anspruch 9, dadurch gekennzeichnet, daß einer von R und R' H, und daß Y CH₃ ist.

11. Medium nach Anspruch 9, dadurch gekennzeichnet, daß einer der Reste R¹ und R² 2-Butyl, 2-Chlor-4-methylvaleryloxy oder 2-Chlor-3-methylvaleryloxy bedeutet.

12. Medium nach einem der Ansprüche 4-11, enthaltend 0,1-50 Gew.% an einer oder mehreren Verbindungen der Formel I.

13. Medium nach mindestens einem der Ansprüche 4-12, dadurch gekennzeichnet, daß es mindestens eine Verbindung der Formel lla 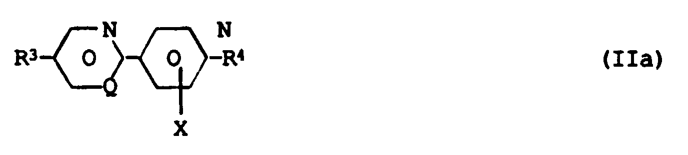 enthält, worin R³ und R⁴ jeweils unabhängig voneinander Alkyl oder Alkoxy mit 5 bis 12 C-Atomen, Q CH oder N und X H oder F bedeutet.

14. Medium nach Anspruch 13, dadurch gekennzeichnet, daß Q N und X H bedeutet.

15. Medium nach Anspruch 13 oder 14, dadurch gekennzeichnet, daß R³ und R⁴ geradkettiges Alkyl bedeuten.

16. Medium nach Anspruch 13, dadurch gekennzeichnet, daß es 35 bis 75 % Phenylpyridine enthält.

17. Medium nach mindestens einem der Ansprüche 4-14, dadurch gekennzeichnet, daß es mindestens eine Verbindung der Formel llb 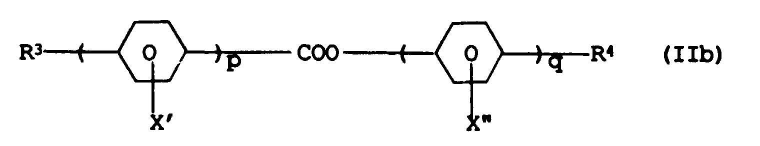 enthält, worin R³ und R⁴ jeweils unabhängig voneinander Alkyl oder Alkoxy mit 5 bis 12 C-Atomen, X' und X" jeweils unabhängig voneinander H oder F und p und q jeweils 1 oder 2 bedeuten, mit der Maßgabe, daß (p + q) 2 oder 3 ist.

18. Medium nach Anspruch 17, dadurch gekennzeichnet, daß R³ und R⁴ geradkettige Alkyl- oder Alkoxy-Gruppen sind.

19. Medium nach Anspruch 17 oder 18, dadurch gekennzeichnet, daß es 10 bis 75 % der angegebenen Ester enthält.

20. Elektrooptisches Anzeigeelement, dadurch gekennzeichnet, daß es als Dielektrikum ein Medium nach mindestens einem der Ansprüche 4-19 enthält.

21. Verbindungen der Formel I
R¹-A¹-Z¹-A²-(Z²-A³)ₙ-R² (I)
worin mindestens einer der Ringe A¹, A² und A³ die Gruppen Thiazol-2,4-diyl, Thiazol-2,5-diyl oder 1 ,3,4-Thiadiazol-2,5-diyl;
A¹, A², und A³ ansonsten jeweils unabhängig voneinander eine unsubstituierte oder durch Halogen, Nitril und/oder Alkyl ein- oder mehrfach substituierte 1,4-Phenylengruppe, worin auch eine oder mehrere CH-Gruppen durch N ersetzt sein können;
eine 1,4-Cyclohexylengruppe, die gegebenenfalls mit CN substituiert sein kann; die Gruppen 1,4-Bicyclo-(2,2,2)-octylen, Piperidin-1,4-diyl, Naphthalin-2,6-diyl, Decahydronaphthalin-2,6-diyl oder 1,2,3,4-Tetrahydronaphthalin-2,6-diyl;
R¹ und R² jeweils unabhängig voneinander eine Alkylgruppe mit 1-15 C-Atomen;
ein hiervon abgeleiteter organischer Rest, der eine oder mehrere der Gruppen -O-, -S-, -CO-, -O-CO-, -CO-O-, -CO-S-, -S-CO-, -CHHalogen-, -CF₂-, -CHCN-, -CAlkylCN- und/oder und/oder -CH=CH-, -C≡C- enthalten und gegebenenfalls ein optische Aktivität verursachendes asymmetrisches Kohlenstoffatom aufweisen kann; wobei zwei Heteroatome nicht direkt miteinander verknüpft sind, einer der Reste R¹ und R² auch F, Cl, Br, CN, COOH, OH, SH, NH₂, NO₂ oder -NCS;
Z¹ und Z² jeweils unabhängig voneinander -CO-O-, -O-CO-, -CH₂CH₂-, -CH₂-O-, -OCH₂-, -N = N-, -NO = N-, -CH = N- oder eine Einfachbindung; eine der Gruppen Z¹ und Z² auch -CH₂-, -O-, -CO-, -CHCN-, -CHHalogen-, -CH₂CH₂CH₂-, -CH₂-COO-oder -CH₂OCO-
und
n 0, 1, 2 oder 3 bedeutet, mit der Maßgabe, daß mindestens einer der Reste R¹ und R² eine Alkylgruppe mit 4-15 C-Atomen ist, worin mindestens eine CH₂-Gruppe durch -CAlkylCN-ersetzt ist und ggf. eine oder mehrere weitere CH₂-Gruppen durch -O-, -S-, -CO-, -O-CO-, -CO-O-, -CO-S-, -S-CO-, -CHHalogen-, -CF₂-, -CHCN-, -CH =CH- und/oder -C≡C- ersetzt sein können.

22. Thiadiazol-Derivate der Formel 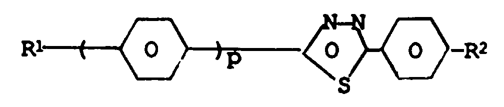 worin p o oder 1,
eine der Gruppen R¹ und R² 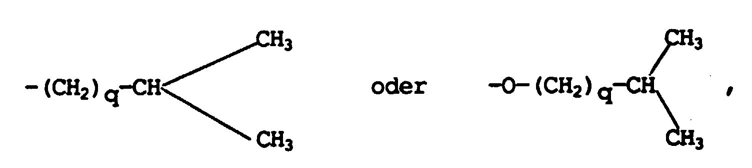 wobei q 2 ibs 7 bedeuten kann, und die andere der Gruppen R¹ und R² geradkettiges Alkyl oder Alkoxy mit 3 bis 12 C-Atomen ist.

23. Thiadiazol-Derivate nach Anspruch 22, dadurch gekennzeichnet, daß q 3 ist.

24. Thiadiazol-Derivae der Formel 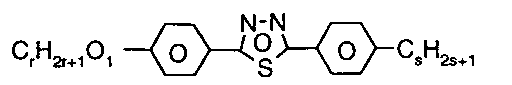 worin r 8, 9 oder 10 und s 5, 6, 7, 8, 9, 10, 11 oder 12 bedeutet, mit der Maßgabe, daß r und s nicht gleichzeitig 10 bedeuten.

25. Thiadiazol-Derivate der Formel 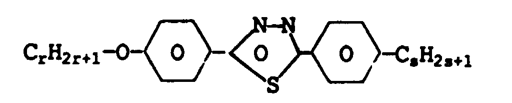 worin r 6 und s 5, 6, 7, 8, 9, 10, 11 oder 12 bedeutet.

26. Thiadiazol-Derivate nach Anspruch 24 oder 25, dadurch gekennzeichnet, daß die Gruppen CᵣH₂ᵣ₊₁ und CₛH₂ₛ₊₁ geradkettige Alkylgruppen sind.

27. Thiadiazol-Derivate nach Anspruch 24 oder 25, dadurch gekennzeichnet, daß r und s wie folgt gewählt werden:
| | | | | | | |
|---|---|---|---|---|---|---|
| r | 6666 | 6 | 8888 | 8 | 9999 | 9 |
| s | 5789 | 10 | 5789 | 10 | 5789 | 10 |

## Claims

1. Use of compounds of the formula I
R¹-A¹-Z¹-A²-(Z²-A³)ₙ-R² (I)
wherein at least one of the rings A¹, A² and A³ is one of the groups 1,3-thiazole-2,4-diyl, 1,3-thiazole-2,5-diyl or 1,3,4-thiadiazole-2,5-diyl,
A¹, A² and A³ otherwise are each independently of one another a 1,4-phenylene group which is unsubstituted or mono- or poly-substituted by halogen, nitrile and/or alkyl and wherein one or more CH groups can also be replaced by N, or a 1,4-cyclohexylene group which can be unsubstituted or CN-substituted, or one of the groups 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl,
R¹ and R² are each independently of one another an alkyl group having 1-15 C atoms, or
an organic radical which is derived therefrom and can contain one or more of the groups -O-, -S-, -CO-, -O-CO-, -CO-O-, -CO-S-, -S-CO-, -CH(halogen)-, -CF₂-, -CHCN-, -C(alkyl)CN- and/or -CH=CH-, -C≡C- and, if appropriate, may have an asymmetric carbon atom causing optical activity, and one of the radicals R¹ and R² can also be F, Cl, Br, CN, COOH, OH, SH, NH₂, NO₂ or -NCS,
Z¹ and Z² are each independently of one another -CO-O-, -OCO-, -CH₂CH₂-, -CH₂-O-, -OCH₂-, -N=N-, -NO=N-, -CH=N- or a single bond, and one of the groups Z¹ and Z² can also be -CH₂-, -O-, -CO-, -CHCN-, -CH(halogen)-, - CH₂CH₂CH₂ - , -CH₂-COO- or -CH₂OCO-,
and
n is 0, 1, 2 or 3,
as components of media having a tilted smectic liquid-crystalline phase, with the provisos that the phase contains 3-15 components, that the phase contains no component in which an H atom is bonded to the carbon atom in the 4-position of the terminal aromatic ring, and that R¹ and R² in the compounds of the formula I are not.

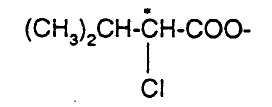

2. Use of compounds of the formula I according to Claim 1 as components of media having a chiral tilted smectic liquid-crystalline phase according to Claim 1.

3. Use according to Claim 1 or 2 of compounds of the formula I, with the exception of trinuclear 1,3,4-thiadiazole-2,5-diyl derivatives in which one of the wing groups R¹ and R² is a chiral radical or both simultaneously are an alkoxy group.

4. Medium which has a tilted smectic liquid-crystalline phase having 3-15 components, at least one of which is a compound of the formula I where R¹ and R² in the compounds of the formula I are not, 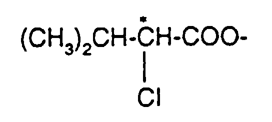 with the proviso that the medium contains no component in which an H atom is bonded to the carbon atom in the 4-position of the terminal aromatic ring.

5. Medium having a chiral tilted smectic liquid-crystalline phase according to Claim 1 and at least two components, characterised in that it contains at least one compound of the formula I.

6. Medium having a chiral tilted smectic C phase according to Claim 1 and at least two components, characterized in that it contains at least one compound of the formula I.

7. Medium according to one of Claims 4-6, containing at least one 3- or 4-nuclear compound of the formula I, wherein one of the bridges Z¹ and Z² is a single bond and the other is -COO-, -OCO- or -CH₂O-.

8. Medium according to one of Claims 4-7, containing at least one compound of the formula 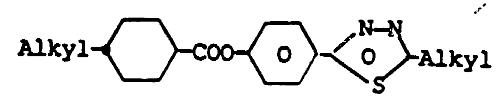 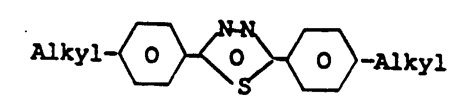 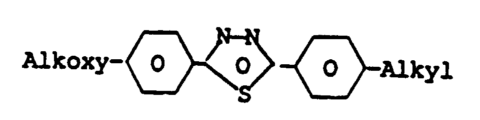 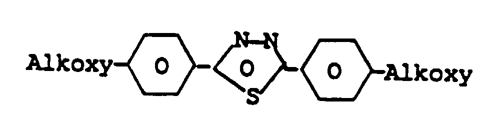 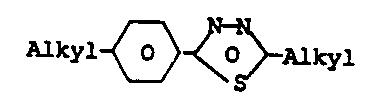 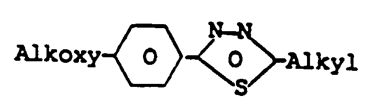 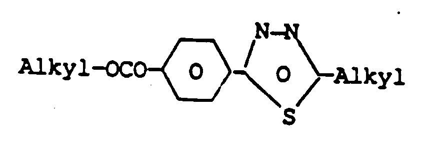 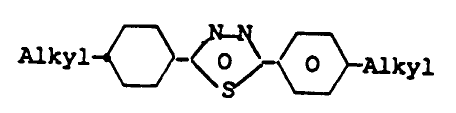 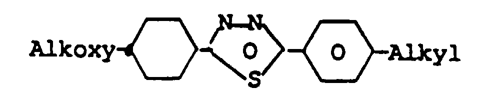 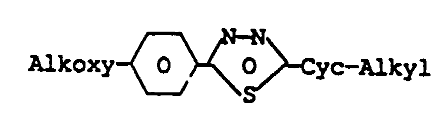 wherein
Alkyl is methyl, ethyl, propyl, butyl, pentyl, hexyl, heptyl, octyl, nonyl or decyl, and
Alkoxy is methoxy, ethoxy, propoxy, butoxy, pentoxy, hexoxy, heptoxy, octoxy, nonoxy or decoxy.

9. Medium according to one of Claims 4-8, containing at least one compound of the formula I wherein one of the radicals R¹ or R² is branched and is represented by 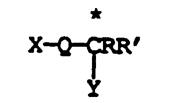 wherein
X is -CO-O, -O-CO-, -O-CO-O-, -CO-, -O-, -S-, -CH=CH-, -CH=CH-COO- or a single bond,
Q is alkylene having 1 to 5 C atoms, wherein a CH₂ group not linked to X can also be replaced by -O-, -CO-, -O-CO-, -CO-O- or -CH=CH-, or a single bond,
Y is CN, halogen, methyl or methoxy, and
R and R' are different from one another and from Y and are each H or an alkyl or alkoxy group which has 1 to 18 C atoms and wherein one or two nonadjacent CH₂ groups can also be replaced by -O-, -CO-, -O-CO-, -CO-O-, and/or -CH=CH-.

10. Medium according to Claim 9, characterized in that one of R and R' is H and that Y is CH₃.

11. Medium according to Claim 9, characterized in that one of the radicals R¹ and R² is 2-butyl, 2-chloro-4-methylvaleryloxy or 2-chloro-3-methylvaleryloxy.

12. Medium according to one of Claims 4-11, containing 0.1-50% by weight of one or more compounds of the formula I.

13. Medium according to at least one of Claims 4-12, characterized in that it contains at least one compound of the formula IIa 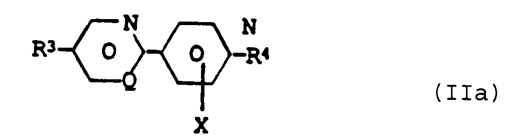 wherein R³ and R⁴ each independently of one another are alkyl or alkoxy having 5 to 12 C atoms, Q is CH or N and X is H or F.

14. Medium according to Claim 13, characterized in that Q is N and X is H.

15. Medium according to Claim 13 or 14, characterized in that R³ and R⁴ are straight-chain alkyl.

16. Medium according to Claim 13, characterized in that it contains 35 to 75% of phenylpyridines.

17. Medium according to at least one of Claims 4-14, characterized in that it contains at least one compound of the formula IIb 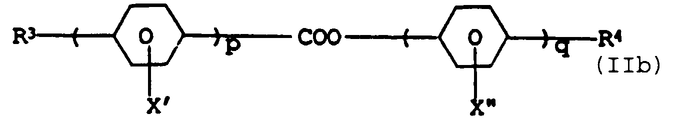 wherein R³ and R⁴ each independently of one another are alkyl or alkoxy having 5 to 12 C atoms, X' and x" each independently of one another are H or F and p and q are each 1 or 2, with the proviso that (p+q) is 2 or 3.

18. Medium according to Claim 17, characterized in that R³ and R⁴ are straight-chain alkyl or alkoxy groups.

19. Medium according to Claim 17 or 18, characterized in that it contains 10 to 75% of the esters indicated.

20. Electro-optical display element, characterized in that it contains a medium according to at least one of Claims 4-19 as the dielectric.

21. Compounds of the formula I
R¹-A¹-Z¹-A²-(Z²-A³)ₙ-R² (I)
wherein at least one of the rings A¹, A² and A³ is one of the groups thiazole-2,4-diyl, thiazole-2,5-diyl or 1,3,4-thiadiazole-2,5-diyl,
A¹, A² and A³ otherwise are each independently of one another a 1,4-phenylene group which is unsubstituted or mono- or poly-substituted by halogen, nitrile and/or alkyl and wherein one or more CH groups can also be replaced by N, or a 1,4-cyclohexylene group which can be unsubstituted or CN-substituted, or one of the groups 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl,
R¹ and R² are each independently of one another an alkyl group having 1-15 C atoms, or an organic radical which is derived therefrom and can contain one or more of the groups -O-, -S-, -CO-, -O-CO-, -CO-O-, -CO-S-, -S-CO-, -CH(halogen)-, -CF₂-, -CHCN-, -C(alkyl)CN- and/or -CH=CH-, -C≡C- and, if appropriate, may have an asymmetric carbon atom causing optical activity, no two heteroatoms being directly linked to one another, and one of the radicals R¹ and R² can also be F, Cl, Br, CN, COOH, OH, SH, NH₂, NO₂ or -NCS,
Z¹ and Z² are each independently of one another -CO-O-, -O-CO-, -CH₂CH₂-, -CH₂-O-, -OCH₂-, -N=N-, -NO=N-, -CH=N- or a single bond, and one of the groups Z¹ and Z² can also be -CH₂-, -O-, -CO-, -CHCN-, -CH(halogen)-, -CH₂CH₂CH₂-, -CH₂-COO- or -CH₂OCO-,
and
n is 0, 1, 2 or 3, with the proviso that at least one of the radicals R¹ and R² is an alkyl group which has 4-15 C atoms and wherein at least one CH₂ group is replaced by -C(alkyl)CN- and, if appropriate, one or more further CH₂ groups can be replaced by -O-, -S-, -CO-, -O-CO-, -CO-O-, -CO-S-, -S-CO-, -CH(halogen)-, -CF₂-, -CHCN-, -CH=CH- and/or -C≡C-.

22. Thiadiazole derivatives of the formula 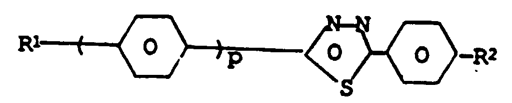 wherein p is 0 or 1,
one of the groups R¹ and R² is 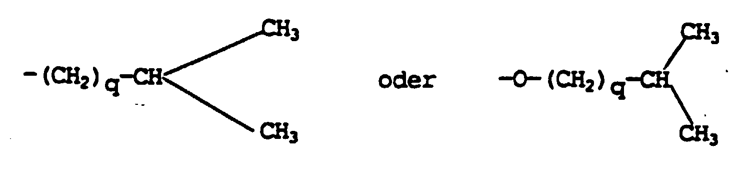 in which q can be 2 to 7, and the other of the groups R¹ and R² is straight-chain alkyl or alkoxy having 3 to 12 C atoms.

23. Thiadiazole derivatives according to Claim 22, characterized in that q is 3.

24. Thiadiazole derivatives of the formula 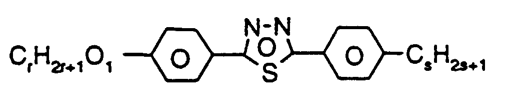 wherein r is 8, 9 or 10 and s is 5, 6, 7, 8, 9, 10, 11 or 12, with the proviso that r and s are not simultaneously 10.

25. Thiadiazole derivatives of the formula 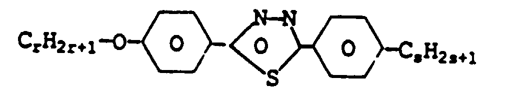 wherein r is 6 and s is 5, 6, 7, 8, 9, 10, 11 or 12.

26. Thiadiazole derivatives according to Claim 24 or 25, characterized in that the groups CᵣH₂ᵣ₊₁ and CₛH₂ₛ₊₁ are straight-chain alkyl groups.

27. Thiadiazole derivatives according to Claim 24 or 25, characterized in that r and s are selected as follows:
| | | | | | | |
|---|---|---|---|---|---|---|
| r | 6666 | 6 | 8888 | 8 | 9999 | 9 |
| s | 5789 | 10 | 5789 | 10 | 5789 | 10 |

## Revendications

1. Utilisation de composés de formule I
R¹-A¹-Z¹-A²-(Z²-A³)ₙ-R² (I)
dans laquelle au moins l'un des cycles A¹, A² et A³ représente le groupe 1,3-thiazole-2,4-diyle, 1,3-thiazole-2,5-diyle ou 1,3,4-thiadiazol-2,5-diyle;
A¹, A² et A³ sinon représentent chacun, indépendamment les uns des autres, un groupe 1,4-phénylène une ou plusieurs fois substitué par un ou des atomes d'halogène ou groupes nitrile et/ou alkyle, dans lequel un ou plusieurs groupes CH peuvent également être remplacés par N,
un groupe 1,4-cyclohexylène qui peut éventuellement être substitué par CN, le groupe 1,4-bicyclo-(2,2,2)-octylène, pipéridine-1,4-diyle, naphtalène-2,6-diyle, décahydronaphtalène-2,6-diyle ou 1,2,3,4-tétrahydronaphtalène-2,6-diyle;
R¹ et R² représentent chacun, indépendamment l'un de l'autre, un groupe alkyle ayant de 1 à 15 atomes de carbone,
un reste organique dérivé de celui-ci, qui peut contenir un ou plusieurs des groupes -O-, -S-, -CO-, -O-CO-, -CO-O-, -CO-S-, -S-CO-, -CH-halogène-, -CF₂-, -CHCN-, -C-alkyl-CN- et/ou -CH=CH-, -C≡C- et éventuellement comporter un atome de carbone asymétrique provoquant l'activité optique; l'un des radicaux R¹ et R² représente également F, Cl, Br, CN, COOH, OH, SH, NH₂, NO₂ ou -NCS;
Z¹ et Z² représentent chacun, indépendamment l'un de l'autre, -CO-O-, -OCO-, -CH₂CH₂-, -CH₂-O-, -OCH₂-, -N=N-, -NO=N-, -CH=N- ou une liaison simple; l'un des groupes Z¹ et Z² représente également -CH₂-, -O-, -CO-, -CHCN-, -CH-halogène-, -CH₂CH₂CH₂-, -CH₂-COO- ou CH₂OCO-
et
n représente 0, 1, 2 ou 3,
en tant que composants de milieux à phase de cristaux liquides smectique inclinée, étant entendu que la phase contient 3-15 composants, que la phase ne contient par de composant dans lequel un atome d'hydrogène est lié à l'atome de carbone on position 4 du cycle aromatique terminal, et que R¹ et R² dans les composés de formule I ne représentent pas

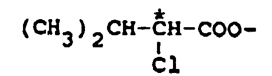

2. Utilisation de composés de formule I selon la revendication 1, en tant que composants de milieux à phase de cristaux liquides smectique inclinés chirale selon la revendication 1.

3. Utilisation selon la revendication 1 ou 2 de composés de formule I à l'exception des dérivés à trois noyaux de 1,3,4-thiadiazole-2,5-diyle, dans lesquels l'un des groupes terminaux R¹ et R² est un reste chiral ou tous les deux représentent simultanément un groupe alcoxy.

4. Milieu comportant une phase de cristaux liquides smectique inclinée chirale à 3-15 composants, dont au moins l'un est un composé de formule I, R¹ et R² dans les composés de formule I ne représentent pas 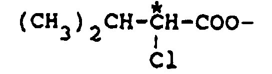 étant entendu que le milieu ne contient pas de composant dans lequel un atome d'hydrogène est lié à l'atome de carbone en position 4 du cycle aromatique terminal.

5. Milieu présentant une phase de cristaux liquides smectique inclinée chirale selon la revendication 1, à au moins deux composants, caractérisé en ce qu'il contient au moins un composé de formule I.

6. Milieu présentant une phase c smectique inclinée chirale selon la revendication 1, à au moins deux composants, caractérisé en ce que celle-ci contient au moins un composé de formule I.

7. Milieu selon l'une des revendications 4 à 6, contenant au moins un composé de formule I à 3 ou 4 noyaux, dans lequel l'un des ponts Z¹ et Z² représente une liaison simple et l'autre représente -COO-,-OCO- ou CH₂O-.

8. Milieu selon l'une des revendications 4 à 7, contenant au moins un composé de formule 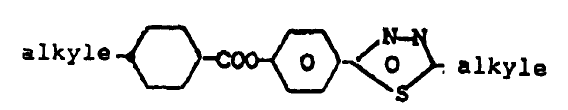 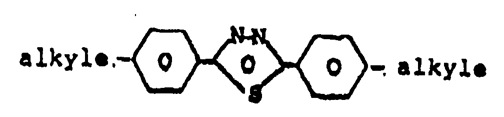 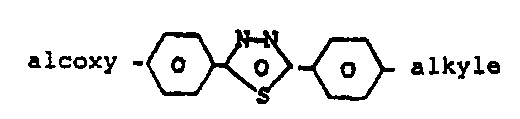 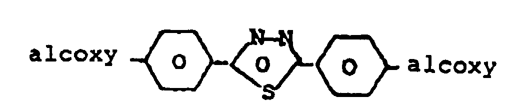 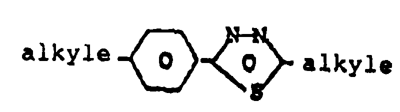 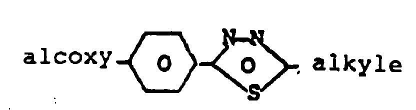 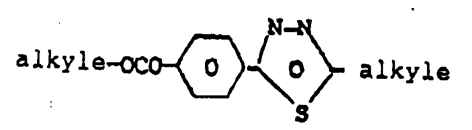 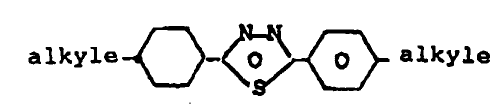 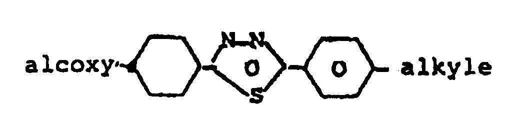 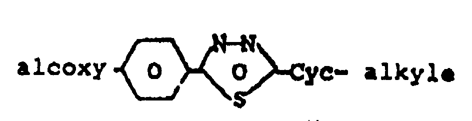 formules dans lesquelles
alkyle représente le groupe méthyle, éthyle, propyle, butyle, pentyle, hexyle, heptyle, octyle, nonyle ou décyle, et
alcoxy représente le groupe méthoxy, éthoxy, propoxy, butoxy, pentoxy, hexoxy, heptoxy, octoxy, nonoxy ou décoxy.

9. Milieu selon l'une des revendications 4 à 8, contenant au moins un composé de formula I dans lequel l'un des radicaux R¹ et R² est ramifié et est représenté par la formule 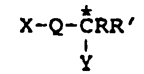 dans laquelle
X représente -CO-O-, -O-CO-, -O-CO-O-, -CO-, -O-, -S-, -CH-CH-, -CH=CH-COO- ou une liaison simple,
Q représente un radical alkylène ayant de 1 à 5 atomes de carbone, dans lequel un croupe CH₂ non lié à X peut également être remplacé par -o-, -CO-, -O-CO-, -CO-O- ou -CH≡CH-, ou une liaison simple,
Y représente un atome d'halogène ou un groupe CN, méthyle ou méthoxy, et
R, R' sont différents l'un de l'autre et de Y, et représentent chacun H ou un radical alkyle ou alcoxy ayant de 1 à 18 atomes de carbone, dans lequel un ou deux groupes CH₂ non contigus peuvent également être remplacés par -O-, -CO-, -O-CO-, -CO-O- et/ou -CH=CH-.

10. Milieu selon la revendication 9, caractérisé en ce que l'un des radicaux R et R' est H et en ce que Y est CH₃.

11. Milieu selon la revendication 9, caractérisé en ce que l'un des radicaux R¹ et R² représente le groupe 2-butyle, 2-chloro-4-méthylvaléryloxy ou 2-chloro-3-méthylvaléryloxy.

12. Milieu selon l'une des revendications 4 à 11, contenant de 0,1 à 50 % en poids d'un ou de plusieurs composés de formule IIa.

13. Milieu selon au moins l'une des revendications 4 à 12, caractérisé en ce qu'il contient au moins un composé de formule IIa 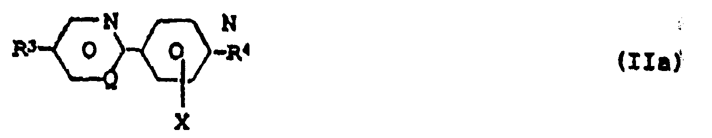 dans laquelle R³ et R⁴ représentent chacun, indépendamment l'un de l'autre, un groupe alkyle ou alcoxy ayant de 5 à 12 atomes de carbone, Q représente CH ou N, et X représente H ou F.

14. Milieu selon la revendication 13, caractérisé en ce que Q représente N et X représente H.

15. Milieu selon la revendication 13 ou 14, caractérisé en ce que R³ et R⁴ représentent chacun un grqupe alkyle à chaîne droite.

16. Milieu selon la revendication 13, caractérisé en ce qu'il contient de 35 à 75 % de phénylpyridine.

17. Milieu selon au moins l'une des revendications 4 à 14, caractérisé en ce qu'il contient au moins un composé de formule IIb 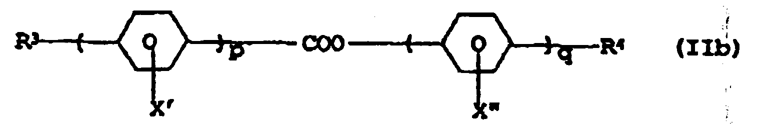 dans laquelle R³ et R⁴ représentent chacun, indépendamment l'un de l'autre, un groupe alkyle ou alcoxy ayant de 5 à 12 atomes de carbone, X' et X" représentent chacun, indépendamment l'un de l'autre, H ou F, et p et q représentent chacun 1 ou 2, étant entendu que (p+q) est égal à 2 ou 3.

18. Milieu selon la revendication 17, caractérisé en ce que R³ et R⁴ sont des groupes alkyle ou alcoxy à chaîne droite.

19. Milieu selon la revendication 17 ou 18, caractérisé en ce qu'il contient de 10 à 75 % des esters indiqués.

20. Elément d'affichage électro-optique, caractérisé en ce qu'il contient comme diélectrique un milieu selon au moins l'une des revendications 4 à 19.

21. Composés de formule I
R¹-A¹-Z¹-A²-(Z²-A³)ₙ-R² (I)
dans laquelle au moins l'un des cycles A¹, A² et A³ repréente le groupe thiazole-2,4-diyle, thiazole-2,5-diyle ou 1,3,4-thiadiazole-2,5-diyle;
A¹, A² et A³ sinon représentent chacun, indépendamment les uns des autres, un groupe 1,4-phénylène une ou plusieurs fois substitué par un ou des atomes d'halogène ou groupes nitrile et/ou alkyle, dans lequel un ou plusieurs groupes CH peuvent également être remplacés par N,
un groupe 1,4-cyclohexylène qui peut éventuellement être substitué par CN, le groupe 1,4-bicyclo-(2,2,2)-octylène, pipéridine-1,4-diyle, naphtalène-2,6-diyle, décahydronaphtalène-2,6-diyle ou 1,2,3,4-tétrahydronaphtalène-2,6-diyle;
R¹ et R² représentent chacun, indépendamment l'un de l'autre, un groupe alkyle ayant de 1 à 15 atomes de carbone,
un reste organique dérivé de celui-ci, qui peut contenir un ou plusieurs des groupes -O-, -S-, -CO-, -O-CO-, -CO-O-, -CO-S-, -S-CO-, -CH-halogène-, -CF₂-, -CHCH-, -C-alkyl-CN- et/ou -CH=CH-, -C≡C- et éventuellement comporter un atome de carbone asymétrique provoquant l'activité optique; l'un des radicaux R¹ et R² représente également F, Cl, Br, CN, COOH, OH, SH, NH₂, NO₂ ou -NCS;
Z¹ et Z² représentent chacun, indépendamment l'un de l'autre, -CO-O-, -OCO-, -CH₂CH₂-, -CH₂-O-, -OCH₂-, -N=N-, -NO=N-, -CH=N- ou une liaison simple; l'un des groupes Z¹ et Z² représente également -CH₂-, -O-, -CO-, -CHCN-, -CH-halogéne-, -CH₂CH₂CH₂-, -CH₂-COO- ou -CH₂OCO-
et
n représente 0, 1, 2 ou 3,
étant entendu qu'au moins l'un des radicaux R¹ et R² est un groupe alkyle ayant de 4 à 15 atomes de carbone, dans lequel au moins un groupe CH₂ peut être remplacé par un groupe -C-alkyl-CN- et un ou plusieurs autres groupes CH₂ peuvent éventuellement être remplacés par -O- -S-, -CO-, -O-CO-, -CO-O-, -CO-S-, -S-CO-, -CH-halogène-, -CF₂-, -CHCN-, -CH=CH- et/ou -C≡C-.

22. Dérivés de thiadiazole de formule 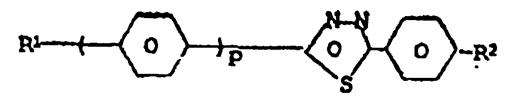 dans laquelle p est 0 ou 1,
l'un des groupes R¹ et R² est 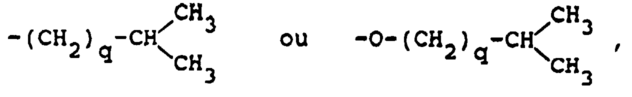 q pouvant représenter 2 à 7, et l'autre des groupes R¹ et R² est un groupe alkyle ou alcoxy à chaîne droite ayant de 3 à 12 atomes de carbone.

23. Dérivés de thiadiazole selon la revendication 22, caractérisés en ce que q est 3.

24. Dérivés de thiadiazole de formule 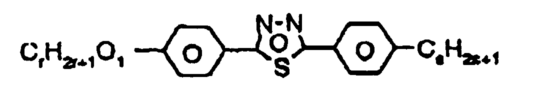 dans laquelle r représente 8, 9 ou 10, et s représente 5, 6, 7, 8, 9, 10, 11 ou 12, étant entendu que r et s ne représentent pas simultanément 10.

25. Dérivés de thiadiazole de formule 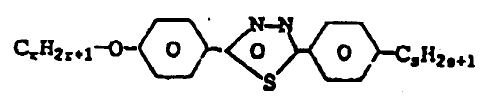 dans laquelle r représente 6 et s représente 5, 6, 7, 8, 9, 10, 11 ou 12.

26. Dérivés de thiadiazole selon la revendication 24 ou 25, caractérisés en ce que les groupes CᵣH₂ᵣ₊₁ et CₛH₂ₛ₊₁ sont des groupes alkyle à chaîne droite.

27. Dérivés de thiadiazole selon la revendication 24 ou 25, caractérisés en ce que r et s sont choisis comme suit:
| | | | | | | |
|---|---|---|---|---|---|---|
| r | 6666 | 6 | 8888 | 8 | 9999 | 9 |
| s | 5789 | 10 | 5789 | 10 | 5789 | 10 |
